# EUROPEAN PATENT SPECIFICATION

(11) **EP 1 802 776 B1**
(45) Date of publication and mention of the grant of the patent: **02.01.2013**
(21) Application number: 05819894.6
(22) Date of filing: 20.10.2005
(51) Int. Cl.: G01N 33/574, C12Q 1/68

(54) **METHOD FOR TAILORING ADMINISTRATION OF DRUGS BY QUANTITATION OF MRNA**
VERFAHREN ZUR ANPASSUNG DER ARZNEIMITTELVERABREICHUNG DURCH QUANTIFIZIERUNG VON MRNA
METHODE PERMETTANT DE PERSONNALISER L'ADMINISTRATION DE MEDICAMENTS PAR ANALYSE QUANTITATIVE DE L'ARNM

(30) Priority: 20.10.2004 US 620603 P; 16.02.2005 US 653557 P; 08.06.2005 US 688741 P
(43) Date of publication of application: 04.07.2007
(73) Proprietor: Hitachi Chemical Company, Ltd., Tokyo 163-0449 (JP); HITACHI CHEMICAL RESEARCH CENTER, INC., Irvine, CA 92617 (US)
(72) Inventor: MITSUHASHI, Masato, Irvine, California 92614 (US)
(74) Representative: Westwood, Joanna
(86) International application number: PCT/US2005/037925
(87) International publication number: WO 2006/045053

(56) References cited:
- US-A1- 2001 006 789
- US-A1- 2003 139 781
- FLORES MONA G ET AL: "In vitro evaluation of the effects of candidate immunosuppressive drugs: flow cytometry and quantitative real-time PCR as two independent and correlated read-outs." JOURNAL OF IMMUNOLOGICAL METHODS JUN 2004, vol. 289, no. 1-2, June 2004 (2004-06), pages 123-135, XP004520885 ISSN: 0022-1759
- KLEIN N J ET AL: "Ex-vivo assessment of candidate anti-inflammatory agents in the treatment of gram negative sepsis" IMMUNOLOGY AND INFECTIOUS DISEASES (OXFORD), vol. 4, no. 1, 1994, pages 33-35, XP008101718 ISSN: 0959-4957
- HAERTEL C ET AL: "Dose-dependent immunomodulatory effects of acetylsalicylic acid and indomethacin in human whole blood: Potential role of cyclooxygenase-2 inhibition" SCANDINAVIAN JOURNAL OF IMMUNOLOGY, vol. 60, no. 4, October 2004 (2004-10), pages 412-420, XP002513851 ISSN: 0300-9475
- FUKUMI SACHIKO ET AL: "Differential responses of Bcl-2 family genes to etoposide in chronic myeloid leukemia K562 cells" MOLECULAR AND CELLULAR BIOCHEMISTRY, vol. 206, no. 1-2, March 2000 (2000-03), pages 43-50, XP002513852 ISSN: 0300-8177
- LIU FENG-TING ET AL: "Bax conformational change is a crucial step for PUMA-mediated apoptosis in human leukemia." BIOCHEMICAL AND BIOPHYSICAL RESEARCH COMMUNICATIONS, vol. 310, no. 3, 24 October 2003 (2003-10-24), pages 956-962, XP004461188 ISSN: 0006-291X
- WYTTENBACH A ET AL: "P53 - Dependent apoptosis induced by DNA damage and its relation to ERK signalling in sympathetic neurones." SOCIETY FOR NEUROSCIENCE ABSTRACT VIEWER AND ITINERARY PLANNER, vol. 2003, 2003, pages Abstract No. 147.3 URL-http://sf, XP008101723 & 33RD ANNUAL MEETING OF THE SOCIETY OF NEUROSCIENCE; NEW ORLEANS, LA, USA; NOVEMBER 08-12, 2003
- CHIARETTI S ET AL: "Gene expression profile of adult T-cell acute lymphocytic leukemia identifies distinct subsets of patients with different response to therapy and survival" BLOOD, AMERICAN SOCIETY OF HEMATOLOGY, US, vol. 103, no. 7, 1 April 2004 (2004-04-01), pages 2771-2778, XP002363618 ISSN: 0006-4971
- HESS D.A. ET AL.: 'The Hydroxylamine of Sulfamethoxazole Synergizes with FK506 and Cyclosporin A, Inhibiting T-Cell Proliferation' THE JOURNAL OF PHARMACOLOGY AND EXPERIMENTAL THERAPEUTICS vol. 281, no. 1, 1997, pages 540 - 548, XP003011828
- BUSH J.A. ET AL.: 'Mini Review, Cancer Chemoresistance: The Relationship Between P53 and Multidrug Transporters' INT. J. CANCER vol. 98, 2002, pages 323 - 330, XP003011830
- SPENCER D.L. ET AL.: 'Quantitative Analysis of Constitutive and 2,3,7,8-Tetrachlorodibenzo-p-dioxin-induced Cytochrome P450 1B1 Expression in Human Lymphocytes' BIOMARKERS & PREVENTION vol. 8, February 1999, pages 139 - 146, XP003011831
- JUHASZ A. ET AL.: 'Quantification of Chemotherapeutic Target Gene mRNA Expression in Human Breast Cancer Biopsies: Comparison of Real-Time Reverse Transcription-PCR vs. Relative Quantification Reverse Transcription-PCR Utilizing DNA Sequences Analysis of PCR Products' JOURNAL OF CLINICAL LABORATORY ANALYSIS vol. 17, 2003, pages 184 - 194, XP003011832

## Description

### BACKGROUND OF THE INVENTION

### Field of the Invention

The present invention relates to a method for tailoring administration of drugs. In the method, whole blood of a patient is exposed to a drug. The level of a marker mRNA linked to an effect of the drug is measured in leukocytes after exposure to the drug and after exposure to a control vehicle only. By comparing the mRNA level after drug exposure with the value after exposure to the control vehicle, or with the value measured before drug exposure, it is possible to determine whether the drug will be effective in the patient. By screening blood of a patient against a number of possible drug remedies, it is possible to develop an optimized treatment protocol tailored to the specific patient.

### Description of the Related Art

The pharmacopoeia offers doctors several possible therapeutic agents for most diseases. However, the efficacy of individual pharmaceutical agents varies substantially from patient to patient. An agent that is, in general, less effective against a particular disease may in fact be highly effective in a particular patient with that disease. It would, therefore, be of great benefit for doctors and patients to be able to determine, in advance, which drug or combination of drugs would be most suitable for each patient, and what individualized doses should be administered to elicit maximal benefit without inducing adverse effects. The ability to make such determinations is commonly termed "tailored medicine," and it is not generally practical at present.

Therapeutic drug monitoring, by measuring drug concentrations in the blood, has been attempted as an initial approach toward tailored medicine. However, the resulting values do not always correlate with the actual effect of the drug in each patient. Another approach has been to rely on genotyping using pharmacogenetics or single nucleotide polymorphism (SNP) information about drug-toxicity-related genes to identify mutations of the genes responsible for drug metabolism. However, such studies, which arm at the discovery of "hot spots" on appropriate genes and the characterization thereof, require a great deal of time and resources. Furthermore, only a limited number of genes linked to drug metabolism have as yet been identified. Moreover, it is not known whether the effect of other as-yet unidentified mutations in the same or other related genes may aggravate or ameliorate the impaired function resulting from the known mutation.

The need to select the appropriate therapeutic regimen is particularly acute in cancer cases, in which the disease can progress quite rapidly if an ineffective protocol is initially selected. There are many anti-cancer drugs available on the market. Since the choice of drugs is based on the type of cancer cells, extensive cytological examinations are conducted to fully characterize individual cancer cells. Patient-to-patient variation exists even within the same cell types, however, and once patients become non-responsive to certain drugs, active therapy is abandoned. If it were possible to screen a wide array of drugs and their combination for efficacy *in vitro,* effective drug regimens could be identified for such patients.

Cancer cells can be isolated from the blood, and maintained in culture media with or without appropriate anti-cancer drugs *in vitro.* Such an assay takes 2-3 days with labor-intensive manipulation steps, however, and these artificial culture conditions often do not reflect drug sensitivity *in vivo.* An assay based on these principles is therefore not applicable as a routine clinical test.

Many anti-cancer pharmaceuticals function by inducing diseased cells to stop progressing through the cell cycle or to enter apoptosis. A gene known to be linked to cell cycle arrest is p21. Furthermore, when cells are promoted to apoptosis, several pro-apoptotic mRNAs are known to be induced. These are the so-called Bcl-2/Bax family genes, which consist of Bax, Bak, Bok, and Bcl-XS, among others. Truncated forms of Bax, such as the so-called BH3-only Bcl-2 family members, are also known to be pro-apoptotic; this group consists of Bid, Bad, Bik, Bim, NOXA, and PUMA (p53 upregulated modulator of apoptosis), among others. These genes share a similar structural motif, which binds to mitochondrial membranes and regulates apoptosis by controlling the release of cytochrome c. Although many pro-apoptotic genes have been identified, we do not know whether all are equally important for the development of apoptosis. Specific genes may be dominantly expressed depending on the type of tissues or cells, or depending on the type or degree of stimuli. Alternatively, the responsible gene may vary among individuals. If a dominant and universal pro-apoptotic mRNA or set of such mRNAs is identified, these will be very useful as common drug targets for cancers and inflammation, where apoptosis plays a crucial role in pathogenesis. Moreover, they will be useful as a universal diagnostic tool for apoptosis-related diseases.

Furthermore, when chemotherapy drugs are employed against solid tumors, one major possible adverse effect is the suppression of leukocytes. In severe cases, this effect can be fatal. However, unfortunately, it is not possible to predict which patients will experience leukocyte suppression, nor when during treatment the effect is likely to occur. Identification of individual patterns of drug response is therefore not only useful for diagnostic purposes, but also for improving the outcomes of clinical trials and avoiding bad publicity by reducing the chance of drug-related death. Improvements in these areas would be of great value to the pharmaceutical industry.

Since the majority of anti-cancer drugs are used intravenously, and blood levels of each drug have been well characterized, one approach is to assess leukocyte toxicity *ex vivo* with cell-based functional assays utilizing known effects of drugs. For example, bleomycin (BLM) is known to induce apoptosis in human lymphocytes via DNA and chromosome breakage. Etoposide (VP-16), a potent topoisomerase II inhibitor, induces DNA strand breaks and apoptosis in human lymphocytes. Although cell-based functional assays are used widely in research, their use in identifying responders and non-responders to individual drugs is not well accepted in clinical practice because of the complexity of such efforts, which involve large variation, and present quantitation difficulties.

A need for tailored medicine is also growing among patients of organ and bone marrow transplantation, because these patients must take immunosuppressants for life. Because cyclosporine A ("CsA") and tacrolimus ("FK") have demonstrated an excellent track record for the management of these patients, these drugs are now used for many other conditions, such as psoriasis, inflammatory bowel diseases, and nephritic syndrome, among others. However, the efficacy of these drugs varies among individual patients, and some show a better response to one of these two drugs. Because the primary action of these drugs is to inhibit transcription of interleukin-2 (IL-2) mRNA, it is reasonable to quantitate the levels of IL-2 mRNA in lymphocytes. However, the methods that have been employed are not suitable as routine clinical tests, mainly because of the difficulty of manipulating the mRNA. The large assay variation is also not suitable for identifying patient-to-patient variation.

The immunosuppressive effects of cylcosporin and tacrolimus have been evaluated in a study on lectin-stimulated whole blood (Flores et al., 2004. In vitro evaluation of the effects of candidate immunosuppressive drugs: flow cytometry and quantitative real-time PCR as two independent and correlated read-outs. J. Immunol. Meth. 289:123-135), while a method for detecting T cell response to specific antigens in whole blood, by either culturing in the presence of a drug or analysing the blood of a subject receiving such a drug is described in US 2001/0006789. The use of a whole blood assay system to compare the efficacy of four drugs to inhibit the release of tumour necrosis factor (TNF) in response to *Escherichia coli* endotoxin 0111:B4 was described in a 1993 study (Klein et al., 1993. Ex-vivo assessment of candidate anti-inflammatory agents in the treatment of gram negative sepsis. Immunol. & Infec. Dis. 4:33-35). Whole blood samples were pre-incubated with acetylsalicylic acid, indomethacin, selective cyclooxygenase (COX)-1 inhibitor (SC-560), COX-2 inhibitor (NS-398) or prostaglandin E2 (PGE2) before stimulation with lipopolysaccharide (LPS) to investigate the dose-dependent immunomodulatory effects (Härtel et al., 2004. Dose-dependent Immunomodulatory Effects of Acetylsalicyclic Acid and Indomethacin in Human Whole Blood: Potential Role of Cyclooxygenase-2 Inhibition. Scand. J. Immunol. 60:412-420). A study carried out in 2000 describes the investigation of dose-dependent effects of etoposide on apoptosis of chronic myeloid leukemia K562 cells (Fukumi et al., 2000. Differential responses of Bcl-2 family genes to etoposide in chronic myeloid leukemia K562 cells. Mol. Cell. Biochem. 206:43-50), while a later study investigated the apoptotic effects of PUMA using the K562 cell line (Liu et al., 2003. Bax conformational change is a crucial step for PUMA-mediated apoptosis in human leukemia. Biochem. Biophys. Res. Comm. 310:956-962).

Summary of the Invention

The present invention discloses a method for tailoring drug protocols to individual patients based on the levels of marker mRNA measured in leukocytes after stimulation of whole blood of the patient with candidate drugs.

One aspect of the invention includes a method of measuring a blood cancer patient's responsiveness to a drug, comprising: exposing heparinized whole blood of the patient to the drug ex vivo for 7 hours or less; exposing heparinized whole blood of the patient to a control vehicle ex vivo for 7 hours or less, after the exposure, measuring the amount of at least one mRNA selected from the group consisting of mRNAs encoding a PUMA gene product and a p21 gene product, both in the blood cells exposed to the drug and in the blood cells exposed to the control vehicle; and identifying responsiveness to the drug by comparing results of the measurement obtained after exposure to the control vehicle with results of the measurement after exposure to the drug, wherein a change in the amount of the mRNA indicates the patient's responsiveness to the drug.

The control vehicle is preferably selected from the group consisting of phosphate-buffered saline and dimethyl sulfoxide.

In a preferred embodiment of the method, the whole blood is stimulated for 5 hours or less. More preferably, the whole blood is stimulated for 2 to 4 hours or less.

In a preferred embodiment of the method, the effect of the drug is apoptosis of blood cells.

In a preferred embodiment of the method, the effect of the drug is cell cycle arrest in blood cells and the mRNA encodes the p21 gene product.

In another preferred embodiment of the method, the amount of a second mRNA associated with a second effect of the drug in blood cells is also measured, and the first effect of the drug is apoptosis of blood cells and the first mRNA encodes the PUMA gene product; and the second effect of the drug is cell cycle arrest in blood cells and the second mRNA encodes the p21 gene product.

In another preferred embodiment of the method, the drug is selected from the group consisting of etoposide, doxorubicin, fludarabine, mitoxantrone, rituximab, vindesine, pirarubicin, carboplatin, cyclophosphamide, bleomycin, vinblastine, vincristine, peplomycin, aclarubicin, daunorubicin, cisplatin, methotrexate, 5-fluorouracil, cytarabine, dacarbazine, cyclophosphamide, and paclitaxel.

In another preferred embodiment of the method, the patient suffers from leukemia or leukemic lymphoma.

In another preferred embodiment of the method, exposing whole blood of the patient includes stimulation with a lectin. More preferably, the lectin is selected from the group consisting of phytohemagglutanin-P and pokeweed mitogen.

Another aspect of the invention includes a method of measuring a blood cancer patient's responsiveness to a drug selected from the group consisting of etoposide, doxorubicin, fludarabine, mitoxantrone, rituximab, vindesine, pirarubicin, carboplatin, cyclophosphamide, bleomycin, vinblastine, vincristine, peplomycin, aclarubicin, daunorubicin, cisplatin, methotrexate, 5-fluorouracil, cytarabine, dacarbazine, cyclophosphamide, and

paclitaxel, comprising: exposing heparinized whole blood of the patient to the drug ex vivo for 4 hours or less; exposing heparinized whole blood of the patient to a control vehicle ex vivo for 4 hours or less; after the exposure, measuring the amount of at least one mRNA selected from the group consisting of mRNAs encoding the p21, and PUMA gene products in blood cells; comparing results of the measurement obtained after exposure to the control vehicle with results of the measurement obtained after exposure to the drug; and identifying responsiveness to the drug based on the results of the comparison, wherein a change in the amount of the mRNA indicates the patient's responsiveness to the drug.

### Brief Description of the Drawings

Figure 1 is a graph showing drug-induced p21 mRNA expression in human blood (open circles), and cultured human leukemic cells.
Figure 2 is a graph showing etoposide-induced p21 mRNA expression from U937 cells mixed in 50 µL of healthy adult blood.
Figure 3 contains bar graphs showing drug-induced p21 (upper) and BAX (lower) mRNA expression in leukemic lymphoma (left, middle) and healthy adult (right).
Figure 4 shows the clinical course of patient 2 from Figure 3.
Figure 5 shows early gene expression during apoptosis after stimulation of heparinized whole blood with 30 Gy radiation (●), 20 µM BLM (▲), 100 µM VP-16 (◆), or corresponding controls ○ (no radiation), Δ (PBS), ◊ (DMSO). Figure 5A shows RNA levels of control, (RNA34) and aragose gel electrophoresis of DNA fragmentation. Lane 1 shows a 100 bp DNA ladder, while lanes 2-6 show 1 day of incubation with DMSO, PBS, VP-16, BLM, and 30 Gy radiation, respectively, and lane 7 shows the results for fresh blood. Figure 5B shows RNA levels of p21 and figure 5C shows RNA levels of PUMA.
Figure 6 shows the results of screening of various pro-apoptotic mRNAs.
Figure 7 shows the results of an assessment of dose response.
Figure 8 shows the results of an assessment of drug-induced gene expression in patients with leukemia and lymphoma.

Figure 9 shows VP-16-induced expression of apoptosis-related genes in human whole blood.

Figure 10 shows BLM-induced expression of apoptosis-related genes in human whole blood.

Figure 11 shows a comparison of p21 and PUMA responses, and VP-16 and BLM responses.

Figure 12 shows day-to-day variation in mRNA level measurements.

Figure 13 shows the taxol-induced expression of apoptosis-related genes in human whole blood.

Figure 14 shows a comparison among drug-induced BAX, p21 and PUMA expression.

Figure 15 shows the BLM-induced p21 mRNA expression in healthy donors and patients with blood cancers.

Figure 16 shows the results of a measurement of p21 and BAX mRNA stimulation in whole blood of cancer patients by a number of anticancer drugs.

Figure 17 shows the results of a measurement of p21, BAX, and PUMA mRNA stimulation in whole blood of cancer patients by a number of anticancer drugs.

Figure 18 is a graph showing the PHA-P-induced induction of leukocyte IL-2 mRNA in heparinized whole blood.

Figure 19 shows the kinetics of PHA-P-induced leukocyte IL-2 mRNA expression in heparinized whole blood.

Figure 20 shows the dose dependent effect of CsA against PHA-P-induced IL-2 mRNA expression.

Figure 21 shows the effect of CsA against PHA-P-induced IL-2 mRNA expression.

Figure 22 shows the responses to 100 and 500 ng/mL CsA on PHA-P-induced IL-2 mRNA expression in healthy donors' blood.

Figure 23 shows the results of a comparison between healthy adults and patients who were taking CsA or FK.

Figure 24A shows the relationship between blood levels of CsA (x-axis) and the values of IL-2 mRNA/number of leukocytes of baseline.

Figure 24B shows the relationship between blood levels of CsA (x-axis) and post-PHA-A stimulation (right).

Figure 25A shows the relationship between blood levels of FK (x-axis) and the values of baseline IL-2 mRNA/number of leukocytes.

Figure 25B shows the relationship between blood levels of FK (x-axis) and the values of IL-2 mRNA/number of leukocytes post-PHA-A stimulation.

### Detailed Description of the Preferred Embodiment

### Embodiment 1: Use of p21/BAX in Tailored Drug Administration For Leukemia and Lymphoma

In the present method, leukocyte cell death resulting from the action of pharmaceutical agents is linked to the transcription level of p21 and BAX mRNA. Of these two marker mRNAs, p21 is responsible for cell cycle arrest, and BAX is induced as an initial signal of apoptosis. If a drug induces p21 in cancer cells, it indicates that the drug exhibits cytostatic activity, whereas BAX induction means that the drug has cytocidal activity. Although many genes are involved during the processes of apoptosis, the expression ot these 2 early gene markers indicate the cytotoxic activities of a drug.

Blood was obtained from healthy adults and from two leukemic non-Hodgkin's malignant lymphoma patients. U937, KG-1, and Jurkat cells were obtained from American Type Culture Collection (ATCC, Manassas, VA), and maintained in RPMI 1640 supplemented with 10% fetal calf serum. Of these, U937 cells are a human histiocytic lymphoma cell line, KG-1 cells are a human bone marrow myelogenous leukemia cell line, and Jurkat cells are a human T-cell lymphoblast-like, leukemia derived cell line. Cells were suspended in serum-free media, and exposed to etoposide (Sigma, St. Louis, MO) for 2 hours at 37°C, then p21 was measured. For human experiments, blood aliquots were stimulated with various anti-cancer drugs at 37°C for 2 hours, then both p21 and BAX mRNA were measured. The anti-cancer drugs used in this study were clinical drugs, and the concentration of each drug was adjusted to typical blood levels 3-6 hours after intravenous administration. The drugs used were Adriacin (doxorubicin, Kyowa Hakko), Fludara (fludarabine, Schering), Novantron (mitoxantron, Wyeth), Vepesid (VP-16, etoposide, Bristol-Myers Squibb), Randa (cisplatin, Nihon Kayaku), Rituxan (rituximab, anti-CD20 monoclonal antibody, Chugai), Fildesin (vindesine, Shionogi), Therarubicin (pirarubicin, Meiji Seika), Paraplatin (carboplatin, Bristol), and Endoxan (cyclophosphamide, Shionogi).

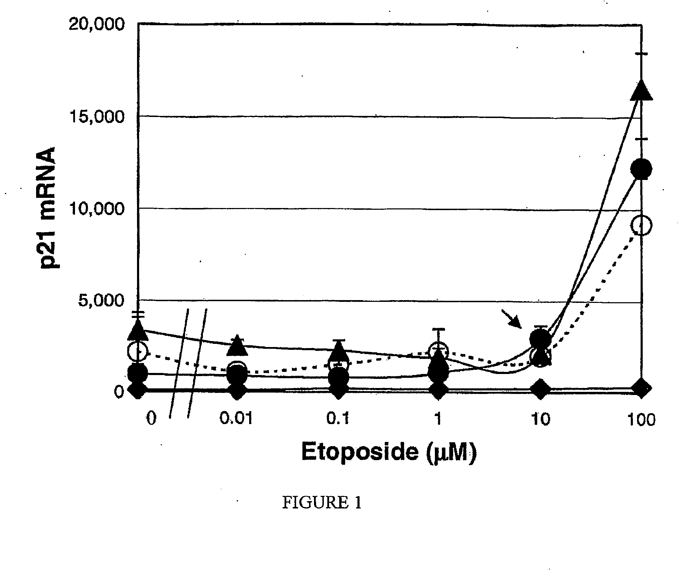

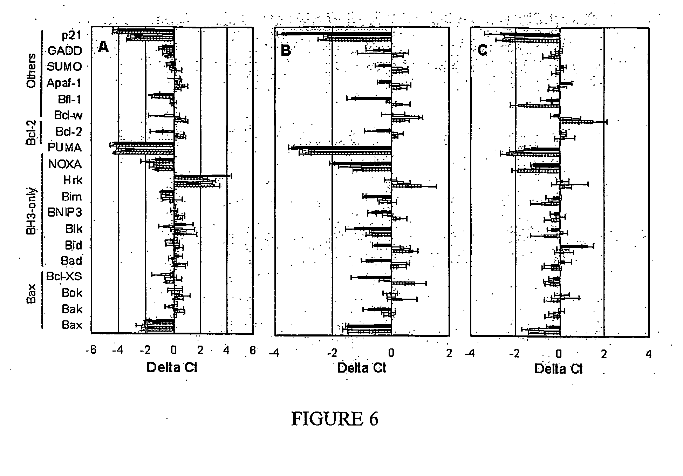

The mRNA and cDNA were prepared from whole blood. In brief, homemade 96-well filterplates were placed over collection plates, and 150 µl 5 mM Tris, pH 7.4, was applied. Following centrifugation at 120 xg for 1 min at 4°C, 50 µl of blood samples were applied to each well and immediately centrifuged at 120 xg for 2 min at 4°C, followed by washing of each well with 300 µl PBS once with centrifugation at 2000 xg for 5 min at 4°C. Then, 60 µl stock lysis buffer, containing for example 0.5% N-Lauroylsarcosine, 4X SSC, 10 mM Tris HCl, pH 7.4, 1 mM EDTA, 0.1% IGEPAL CA-630, and 1.791 M guanidine thiocyanate, supplemented with 1% 2-mercaptoethanol (Bio Rad, Hercules, CA, USA), 0.5 mg/ml proteinase K (Pierce, Rockford, IL, USA), 0.1 mg/ml salmon sperm DNA (5 Prime Eppendorf/Brinkmann, Westbury, NY, USA), 0.1 mg/ml E. coli tRNA (Sigma), a cocktail of 10 mM each of the specific reverse primers shown in Table 1, and standard RNA34 oligonucleotides, were applied to the filterplates, followed by incubation at 37°C for 10 min. The filterplates were then placed over oligo(dT)-immobilized microplates (GenePlate, RNAture), and centrifuged at 2000 xg for 5 min at 4°C. Following overnight storage at 4°C, the microplates were washed with 100 µl plain lysis buffer 3 times, followed by 150 µl of wash buffer (0.5 M NaCl, 10 mM Tris, pH 7.4, 1 mM EDTA) 3 times at 4°C. The cDNA was directly synthesized in each well by adding 30 µl buffer containing 1x RT-buffer, 1.25 mM each of dNTP, 4 units rRNasin, and 80 units of MMLV reverse transcriptase (Promega) (without primers), and incubation at 37°C for 2 hours. The specific primer-primed cDNA existed in solution, and oligo(dT)-primed cDNA stayed immobilized in the microplate. For TaqMan PCR (Figs. 1, 3, 4), the resultant 4 µl cDNA solution was directly transferred to 384-well PCR plates, to which 5 µl of TaqMan universal master mix (ABI) and 1 µl oligonucleotide cocktail (15 µM each of forward and reverse primer, and 3-6 µM TaqMan probe) were applied, and PCR was conducted in PRISM 7900HT (ABI), with one cycle of 95°C for 10 min followed by 45 cycles of 95°C for 30 sec, 55°C for 30 sec, and 60°C for 1 min. For SYBR Green PCR (see Fig. 6), cDNA was diluted 3-4 fold in water, and 4 µl cDNA solution was directly transferred to 384-well PCR plates, to which 5 µl of a master mix (BioRad, Hercules, CA) and 1 µl of oligonucleotide cocktail (15 µM each of forward and reverse primer) were applied, and PCR was conducted in PRISM 7900HT (ABI), with one cycle of 95°C for 10 min followed by 45 cycles of 95°C for 30 sec and 60°C for 1 min. Each gene was amplified in separate wells. The Ct was determined by analytical software (SDS, ABI).

In order to determine the appropriate concentration of etoposide to employ in order to induce p21 mRNA in blood and in cultured cells, blood samples from healthy adults were stimulated with various concentrations of the drug at 37°C for 2 hours, then the levels of p21 mRNA were determined. As shown in Fig. 1, open circles, no significant p21 mRNA expression was detected in healthy leukocytes at 10 µM etoposide, although p21 was expressed significantly at 100 µM. In contrast, however, slight but significant p21 expression was detected in U937 cells (Fig. 1, closed circles, arrow), when the cells were exposed to 10 µM etoposide. The drug sensitivity of KG-1 cells (Fig. 1, closed triangles) was similar to that of human blood, and Jurkat cells were resistant to etoposide even when 100 µM was used (Fig. 1, closed diamonds). These results indicate that p21 mRNA expression was a useful marker for drug cytotoxicity, and 10 µM etoposide is a window for the selection of sensitive cancer cells.

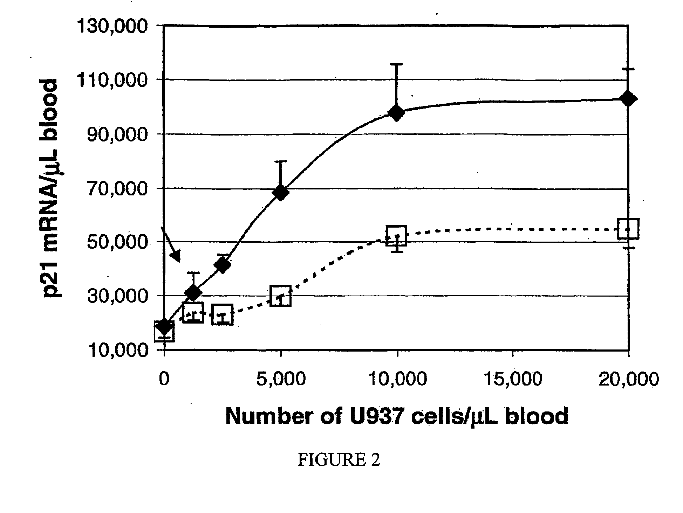

The results shown in Fig. 1 indicated that 10 µM etoposide was a cutoff value for the differentiation between healthy blood and U937. In order to simulate leukemia using cultured cells, various amounts of U937 were mixed with blood, and exposed to 10 µM etoposide. The results are shown in Figure 2. In order to avoid blood-to-U937 interactions, etoposide was exposed to blood and cells in separate tubes, and applied to the same wells of filterplates. In order to identify native p21 mRNA expression in U937, the same amounts of untreated U937 were also added to blood samples as a control. In the Figure, open squares indicate DMSO-treated U937 cells, and closed diamonds indicate 10 µM etoposide-treated U937 cells. As shown in Fig. 2, significant (p=0.04) p21 mRNA expression was identified when 1,250 U937 cells were added to 1 µL blood (arrow). These results indicate that the drug sensitivity of cancer cells can be identified in whole blood when cancer cell population is as small as 20% (1,250 cancer cells per 5,000 normal leukocytes per 1 µL blood).

The cultured cell conditions that produced the results shown in Figs. 1-2 were artificial, and do not necessarily reflect drug action *in vivo.* Results from cultured cells can be difficult to interpret. In contrast, data derived from whole blood result from conditions that are very close to physiological conditions, and such data are easy to interpret.

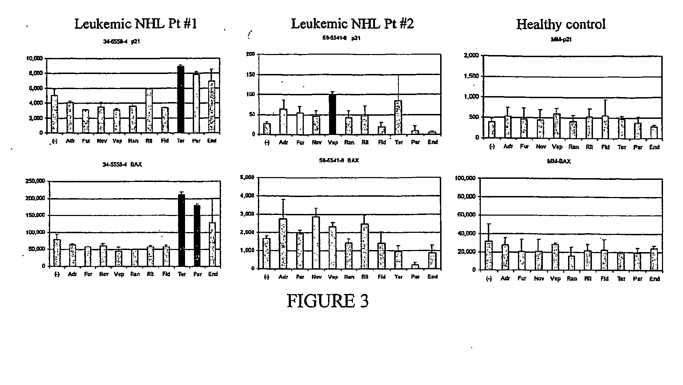

Fig. 3 shows the results of drug-induced p21 and BAX mRNA expression in 2 leukemic lymphoma patients and 1 healthy adult in response to a number of drugs. In the Figure, Adr indicates Adriacin (400 nM), Fur indicates Fludara (0.2 µg/ml), Nov indicates Novantron (10 ng/ml), Vep indicates Vepesid (5 µg/ml), Ran indicates Randa (cisplatin) (1 µg/ml), Rit indicates Rituxan (200 µg/ml), Fid indicates Fildesin (10 ng/ml), Ter indicates Therarubicin (50 ng/ml), Par indicates Paraplatin (5 µg/ml), and End indicates Endoxan (1 µg/ml). These concentrations were determined based on blood levels 1-4 hours after intravenous injection. The black bars indicate p<0.001, while light gray bars indicate p<0.05.

Although all drugs failed to induce p21 and BAX mRNA in the healthy adult, patient 1 showed significant induction of both p21 and BAX mRNA when blood was stimulated with Therarubicin and Paraplatin. Endoxan also induced p21 mRNA. Patient 2 showed significant p21 mRNA induction by Adriacin and Fludara, and BAX mRNA induction by Novantron. Vepsid induced both p21 and BAX mRNA in patient 2. Since the population of atypical lymphocytes in both patients was more than 80%, these results were derived from malignant cells, even though whole blood was tested. The results shown in Fig. 3 indicate that 1) the method is sensitive enough to identify cancer-cell-derived gene expression in whole blood, and 2) drug responsiveness varied widely, even though the cancer cells in these patients were of B-cell origin.

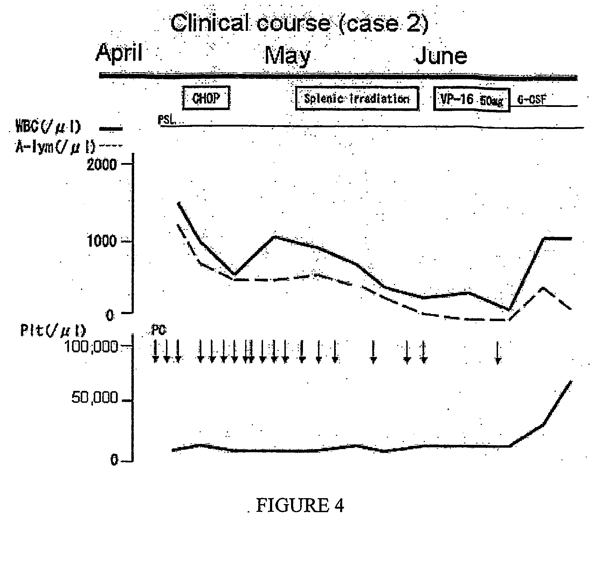

Figure 4 shows the further clinical course of Patient 2, who failed to respond to CHOP chemotherapy (cyclophosphamide (Endoxan), doxorubicin (Adriacin), vincristin, and predonisolone) and subsequent splenic radiation, and showed continued thrombocytopenia. However, as shown in the Figure the platelet count increased substantially when Vepesid (VP-16, etoposide) was administered. Since granulocyte colony-stimulating factor (G-CSF) has no function against platelets, the improvement of thrombocytopenia is ascribable to the action of etoposide. Since etoposide was the only drug which induced both p21 and BAX mRNA in patient 2, these clinical results indicate that the measurement of drug-induced p21 and BAX mRNA expression in whole blood will be useful in the identification of sensitive drugs for each patient.

In each case, sensitive drugs were identified by measuring drug-induced p21 and BAX mRNA expression in whole blood. Since the assay variation was very small and the starting materials were triplicate aliquots of whole blood, the results were statistically significant. Interestingly, the list of sensitive drugs differed between the two patients, although both patients had non-Hodgkin's lymphoma with B-cell characteristics. The selection of these drugs cannot be accomplished without this mRNA test. Therefore, this test will be a powerful tool for tailored medicine for blood cancers.

Unlike solid tumors, blood cancer cells exist in the blood. Thus, while the maximally tolerable drug concentration was determined for each drug in whole blood in terms of p21 and BAX mRNA induction (Fig. 3, right panels), the drug sensitivity for cancer cells can be identified by simply mixing candidate drugs in whole blood (Fig. 3, left and middle panels). Moreover, it was necessary to incubate the drugs for only 2 hours, which eliminates potential secondary effects. Although it is possible that the apoptosis cascade may be blocked downstream after p21 and BAX expression, drugs that induce p21 and BAX expression are more likely to be efficacious than other drugs which failed to induce the mRNA of these genes. The fact that the second patient in this embodiment responded to the drug that was identified (Fig. 4) shows that the present method will be useful in tailored medicine for cancer chemotherapy in the future.

### Embodiment 2: Use of p21 and PUMA mRNA in Tailored Drug Administration For Leukemias and Lymphoma

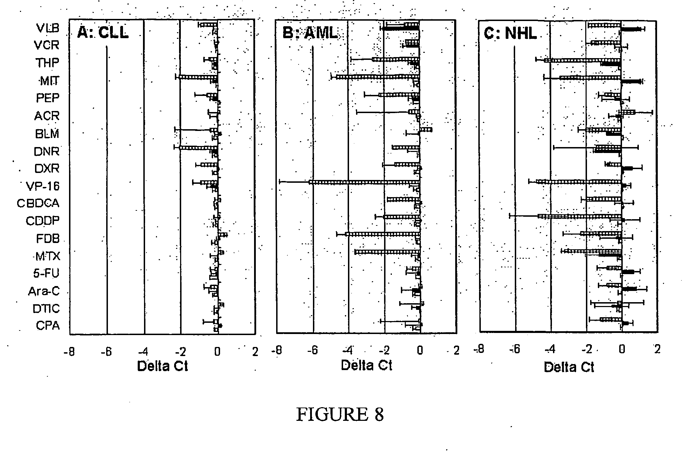

PUMA or Bcl-2 binding component 3 (bbc3) was discovered by 3 independent groups at the almost same time in 2001, and given GenBank accession numbers HSU82987, AF332558, and AF354656, respectively. According to GenBank information, these sequences were submitted in December 1996, December 2000, and March 2001, respectively, and the oldest entry, UniGene (Hs.467020) used bb3 as the title of this gene. Many publications use PUMA (p53 regulated modulator of apoptosis), not bbc3. It is not universally expressed in all types of cells, and according to the expression profile data in UniGene (Hs.467020), it is expressed in blood, cervix, colon, eye, kidney, larynx, lung, mammary gland, ovary, skin, small intestine, stomach, and testis. PUMA has also been reported to be a major mediator of drug-induced apoptosis in mice. Although PUMA was characterized extensively in each experimental system, no link has yet been established between PUMA and other pro-apoptotic mRNAs in human blood leukocytes. Blood is particularly important, because detection of an early apoptosis signal in blood is expected to lead to new diagnostic developments for the identification of effective anti-cancer drugs for each blood disease patient (see Fig. 8). Moreover, any anti-cancer drug for solid tumors induces some adverse effects on leukocytes, which are sometimes fatal. Thus, individual drug toxicity may be predicted by *ex vivo* incubation of blood and target drugs. Identification of responders or non-responders to certain drugs is believed to be useful for the processes of drug development.

Drug sensitivity tests are usually conducted using isolated mononuclear leukocytes, which are suspended in culture media and incubated for a couple of days in a CO₂ incubator to confirm the establishment of cell death or apoptosis, or equivalent biological indicators. Since these conditions are very different from native conditions, the results are difficult to interpret. This is one of the main reasons why drug sensitivity tests are not common in clinical practice, even though the identification of suitable drugs is essential for patients' quality of life. The present method avoids this problem, by using whole blood and a short 2-4 hours' incubation. The present method allows the absolute quantity of any given mRNA to be sensitively determined from as little as 50 µl of human whole blood. Since the high throughput platform permits the use of triplicate whole blood samples, results are reliable with a reasonable statistical analysis. Genotyping is the current new trend for drug sensitivity assays; however, it is difficult to reach a conclusion based on genetic polymorphisms or mutations in one or more locations of a certain gene, because it is not known whether genetic polymorphisms or mutations in other genes may compensate for the first abnormalities. The present method provides a phenotypic test using genetics as a tool, and avoids these problems.

When significant drug-induced p21 and/or PUMA mRNA induction is detected by this system, these positive results indicate that the drugs are at least functional to initiate an early apoptosis cascade under physiological *ex vivo* conditions. Drugs exhibiting a positive result in this test are more likely to be good candidates for therapy than those exhibiting negative results. The final establishment of apoptosis in each blood sample can be confirmed using an assay having a shorter incubation period, so as to more reliably replicate physiological conditions. Since p21 is responsive for cell cycle arrest, and PUMA is pro-apoptotic, analysis of these genes will correspond to cytostatic and cytocidal effects of drugs.

The following methods were employed in carrying out the method of the present invention.

Blood samples were obtained from healthy adult volunteers and from disease patients. Various Bax-related genes as well as genes which were reported to be induced during apoptosis were identified through a literature search, and corresponding PCR primers and TaqMan probes were designed by Primer Express (Applied Biosystem, Foster City, CA) and HYBsimulator (RNAture, Irvine, CA). Oligonucleotides were synthesized by IDT (Coralville, IA). The GenBank accession numbers and primer sequences are summarized in Table 1 below.

**TABLE 1: GENE PRIMER SEQUENCES**

| | GeneBank | Sequences (5'-3') | |
|---|---|---|---|
| mRNA | Accession # | Forward primer | Reverse primer |
| p21 | HS431A14 | TTCTGCTGTC TCTCCTCAGA TTTCT (SEQ ID NO: 1) | GGATTAGGGC TTCCTCTTGG A (SEQ ID NO: 2) |

| TagMan probe: FAM-CCACTCCAAA CGCCGGCTGA TC-TAMRA (SEQ ID NO: 3) | | | |
|---|---|---|---|
| GADD153 | S40706 | AGAACCAGGA AACGGAAACA GA (SEQ ID NO: 4) | TCTCCTTCAT GCGCTGCTTT (SEQ ID NO: 5) |
| SUMO-1 | BC006462 | GGGTCAGAGA ATTGCTGATA ATCAT (SEQ ID NO: 6) | CCCCGTTTGT TCCTGATAAA CT (SEQ ID NO: 7) |
| Apaf-1 | AF013263 | TGCGCTGCTC TGCCTTCT (SEQ ID NO: 8) | CCATGGGTAG CAGCTCCTTC T (SEQ ID NO:9) |
| Bfl-1 | U27467 | CACAGGAGAA TGGATAAGGC AAA (SEQ ID NO:10) | CATCCAGCCA GATTTAGGTT CAA (SEQ ID NO:11) |
| Bcl-w | NM_004050 | TCCGGCGCAC CTTCTCT (SEQ ID NO:12) | CCCAAAGACA AAGAAGGCTA CAA (SEQ ID NO:13) |
| Bcl-2 | BC027258 | CATGTGTGTG GAGAGCGTCA A (SEQ ID NO: 14) | GCCGGTTCAG GTACTCAGTC A (SEQ ID NO: 15) |
| PUMA | AF354654 | GGGCCCAGAC TGTGAATCCT (SEQ ID NO: 16) | ACGTGCTCTC TCTAAACCTA TGCA (SEQ ID NO: 17) |

| TagMan probe: FAM- CCCCGCCCCA TCAATCCCA (SEQ ID NO: 18) | | | |
|---|---|---|---|
| NOXA | BC032663 | CTCAGGAGGT GCACGTTTCA (SEQ ID NO: 19) | TTCCAAGGGC ACCCATGA (SEQ ID NO: 20) |
| HRK | HSU76376 | GGGAGCCCAG AGCTTGAAA (SEQ ID NO: 21) | GCGCTGTCTT TACTCTCCAC TTC (SEQ ID NO: 22) |
| BIM | BC033694 | TCCAGGACAG TTGGATATTG TCA (SEQ ID NO: 23) | TAAGGAGCAG GCACAGAGAA AGA (SEQ ID NO: 24) |
| BNIP3 | BC021989 | AAATATTCCC CCCAAGGAGT TC (SEQ ID NO: 25) | CGCTCGTGTT CCTCATGCT (SEQ ID NO: 26) |
| BIK | U34584 | TCCTATGGCT CTGCAATTGT CA (SEQ ID NO: 27) | GGCAGGAGTG AATGGCTCTT C (SEQ ID NO: 28) |
| BID | BC036364 | CATACACTTT TTCTCTTTCC ATGACATC (SEQ ID NO: 29) | GGGCATCGCA GTAGCTTCTG (SEQ ID NO: 30) |
| BAD | BC001901 | CAGGCCTATG CAAAAAGAGG AT (SEQ ID NO: 31) | CGCACCGGAA GGGAATCT (SEQ ID NO: 32) |
| Bcl-Xs | NM_001191 | GGCAGGCGAC GAGTTTGA (SEQ ID NO: 33) | GTTCCCATAG AGTTCCACAA AAGTATC (SEQ ID NO: 34) |
| BOK | NM_032515 | TCACATGCTG GTTGCTTAAT CC (SEQ ID NO: 35) | GCACAAGGAC CCCATCACA (SEQ ID NO: 36) |
| BAK | NM_001188 | CACGGCAGAG AATGCCTATG A (SEQ ID NO: 37) | CCCAATTGAT GCCACTCTCA (SEQ ID NO: 38) |
| BAX | AY217036 | TTTCTGACGG CAACTTCAAC TG (SEQ ID NO: 39) | GGTGCACAGG GCCTTGAG (SEQ ID NO: 40) |

In 8-well strip microtubes, 1.4 µl of 50x concentrations of drugs or controls (phosphate buffered saline (PBS) or DMSO) were added, and stored at -20°C until use. The chemicals used were vinblastine (VLB), vincristine (VCL), mitoxantrone (MIT), aclarubicin (ACR), bleomycin (BLM), daunorubicin (DNR), doxorubicin (DXR), etoposide (VP-16), carboplatin (CBDCA), cisplatin (CDDP), Fludarabine (FDB), methotrexate (MTX), 5-fluorouracil (5-FU), cytarabine (Ara-C), dacarbazine (DTIC), cyclophosphamide (CPA) (from Sigma, St Louis, MO), pirarubicin (THP), and peplomycin (PEP) (from Wako Pure Chemicals, Osaka, Japan). Seventy µl of fresh heparinized whole blood was added into each well in triplicate, and incubated at 37°C for 2-8 hours with the cap closed. For radiation treatment, blood was stimulated at designated doses using cesium-137. After each treatment, 50 µl of whole blood was transferred to filterplates as described below.

The mRNA and cDNA were prepared from whole blood. In brief, homemade 96-well filterplates were placed over collection plates, and 150 µl 5 mM Tris, pH 7.4, was applied. Following centrifugation at 120 xg for 1 min at 4°C, 50 µl of blood samples were applied to each well and immediately centrifuged at 120 xg for 2 min at 4°C, followed by washing of each well with 300 µl PBS once with centrifugation at 2000 xg for 5 min at 4°C. Then, 60 µl stock lysis buffer, containing for example 0.5% N-Lauroylsarcosine, 4X SSC, 10 mM Tris HCl, pH 7.4, 1 mM EDTA, 0.1% IGEPAL CA-630, and 1.791 M guanidine thiocyanate, supplemented with 1% 2-mercaptoethanol (Bio Rad, Hercules, CA, USA), 0.5 mg/ml proteinase K (Pierce, Rockford, IL, USA), 0.1 mg/ml salmon sperm DNA (5 Prime Eppendorf/Brinkmann, Westbury, NY, USA), 0.1 mg/ml E. coli tRNA (Sigma), a cocktail of 10 mM each of the specific reverse primers shown in Table 1, and standard RNA34 oligonucleotides, were applied to the filterplates, followed by incubation at 37°C for 10 min. The filterplates were then placed over oligo(dT)-immobilized microplates (GenePlate, RNAture), and centrifuged at 2000 xg for 5 min at 4°C. Following overnight storage at 4°C, the microplates were washed with 100 µl plain lysis buffer 3 times, followed by 150 µl of wash buffer (0.5 M NaCl, 10 mM Tris, pH 7.4, 1 mM EDTA) 3 times at 4°C. The cDNA was directly synthesized in each well by adding 30 µl buffer containing 1x RT-buffer, 1.25 mM each of dNTP, 4 units rRNasin, and 80 units of MMLV reverse transcriptase (Promega) (without primers), and incubation at 37°C for 2 hours. The specific primer-primed cDNA existed in solution, and oligo(dT)-primed cDNA stayed immobilized in the microplate. For TaqMan PCR (Figs. 1, 3, 4), the resultant 4 µl cDNA solution was directly transferred to 384-well PCR plates, to which 5 µl of TaqMan universal master mix (ABI) and 1 µl oligonucleotide cocktail (15 µM each of forward and reverse primer, and 3-6 µM TaqMan probe) were applied, and PCR was conducted in PRISM 7900HT (ABI), with one cycle of 95°C for 10 min followed by 45 cycles of 95°C for 30 sec, 55°C for 30 sec, and 60°C for 1 min. For SYBR Green PCR (see Fig. 6), cDNA was diluted 3-4 fold in water, and 4 µl cDNA solution was directly transferred to 384-well PCR plates, to which 5 µl of a master mix (BioRad, Hercules, CA) and 1 µl of oligonucleotide cocktail (15 µM each of forward and reverse primer) were applied, and PCR was conducted in PRISM 7900HT (ABI), with one cycle of 95°C for 10 min followed by 45 cycles of 95°C for 30 sec and 60°C for 1 min. Each gene was amplified in separate wells. The Ct was determined by analytical software (SDS, ABI). Although 1x RT buffer was used as a negative control, none of the primer pairs produced non-specific primer-dimers under these experimental conditions. For PUMA in Fig. 8, immobilized cDNA on the oligo(dT)-immobilized microplates was directly amplified in an iCycler (BioRad).

Using a commercial kit (Puregene, Gentra, Minneapolis, MN), genomic DNA was purified from 300 µl each of whole blood with or without treatments. DNA was then analyzed in 3.5% agarose (3:1 NuSieve:agarose, FMC, Rockland, ME) gel electrophoresis, stained with 0.5 µg/ml ethidium bromide, and photographic images were recorded by AlphaImager (Alpha Innotech, San Leandro, CA).

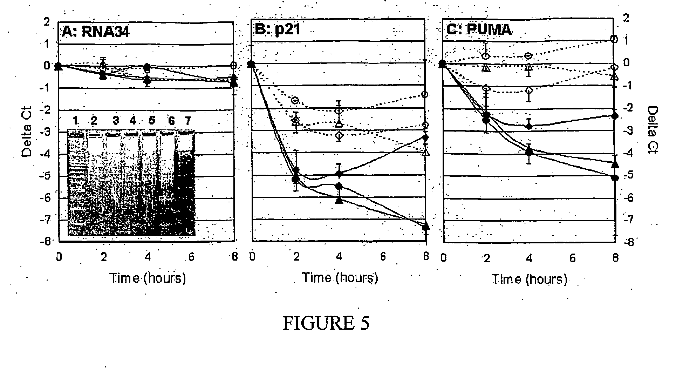

In order to induce apoptosis, heparinized human whole blood was stimulated with 30 Gy of ionizing radiation, 20 µM bleomycin (BLM), or 100 µM etoposide (VP-16), respectively. 15-25 Gy of radiation is clinically used to kill donor leukocytes to prevent graft-versus-host disease during blood transfusion. Bleomycin is known to induce apoptosis in human lymphocytes via DNA and chromosome breakage. Etoposide, a potent topoisomerase II inhibitor, induces DNA strand breaks and apoptosis in human lymphocytes. To mimic physiological conditions, whole blood was used, without isolating mononuclear cells. Fragmentation of the DNA, a typical sign of apoptosis, was analyzed as shown m the mset in Figure 5. After 1 day of incubation, genomic DNA was extracted, and DNA fragmentation was analyzed by agarose gel electrophoresis using ethidium bromide staining. Lane 1 shows a 100 bp DNA ladder (smallest shown: 200 bp, Invitrogen, CA), while lanes 2-6 show 1 day of incubation with DMSO, PBS, 100 µM VP-16, 20 µM BLM, and 30 Gy radiation, respectively, and lane 7 shows the results for fresh blood ("Cont" in the Figure). As shown in the Figure, fragmentation of DNA was observed.

Quantitation of various mRNAs was conducted using the following method. Triplicate aliquots of 50 µl each of heparinized whole blood was applied to 96-well filterplates to trap leukocytes. The filterplates were placed over collection plates, and 150 µl 5 mM Tris, pH 7.4, was applied. Following centrifugation at 120 xg for 1 min at 4°C, 50 µl of blood samples were applied to each well and immediately centrifuged at 120 xg for 2 min at 4°C, followed by washing of each well with 300 µl PBS once with centrifugation at 2000 xg for 5 min at 4°C. Then, 60 µl stock lysis buffer, containing for example 0.5% N-Lauroylsarcosine, 4X SSC, 10 mM Tris HCl, pH 7.4, 1 mM EDTA, 0.1% IGEPAL CA-630, and 1.791 M guanidine thiocyanate, supplemented with 1% 2-mercaptoethanol (Bio Rad, Hercules, CA, USA), 0.5 mg/ml proteinase K (Pierce, Rockford, IL, USA), 0.1 mg/ml salmon sperm DNA (5 Prime Eppendorf/Brinkmann, Westbury, NY, USA), 0.1 mg/ml E. coli tRNA (Sigma), a cocktail of 10 mM each of the specific reverse primers shown in Table 1, and standard RNA34 oligonucleotides, were applied to the filterplates, followed by incubation at 37°C for 10 min. The filterplates were then placed over oligo(dT)-immobilized microplates (GenePlate, RNAture), and centrifuged at 2000 xg for 5 min at 4°C. Following overnight storage at 4°C, the microplates were washed with 100 µl plain lysis buffer 3 times, followed by 150 µl wash buffer (0.5 M NaCl, 10 mM Tris, pH 7.4, 1 mM EDTA) 3 times at 4°C. The cDNA was directly synthesized in each well by adding 30 µl buffer containing 1x RT-buffer, 1.25 mM each of dNTP, 4 units rRNasin, and 80 units of MMLV reverse transcriptase (Promega) (without primers), and incubation at 37°C for 2 hours. The specific primer-primed cDNA existed in solution, and oligo(dT)-primed cDNA stayed immobilized in the microplate. For TaqMan PCR (Figs. 1, 3, 4), the resultant 4 µl cDNA solution was directly transferred to 384-well PCR plates, to which 5 µl TaqMan universal master mix (ABI) and 1 µl oligonucleotide cocktail (15 µM each of forward and reverse primer, and 3-6 µM TaqMan probe) were applied, and PCR was conducted in PRISM 7900HT (ABI), with one cycle of 95°C for 10 min followed by 45 cycles of 95°C for 30 sec, 55°C for 30 sec, and 60°C for 1 min. For SYBR Green PCR (Fig. 2), cDNA was diluted 3-4 fold in water, and 4 µl cDNA solution was directly transferred to 384-well PCR plates, to which 5 µl of a master mix (BioRad, Hercules, CA) and 1 µl of oligonucleotide cocktail (15 µM each of forward and reverse primer) were applied, and PCR was conducted in PRISM 7900HT (ABI), with one cycle of 95°C for 10 min followed by 45 cycles of 95°C for 30 sec and 60°C for 1 min. Each gene was amplified in separate wells. The PCR cycle where certain amounts of PCR products were generated was determined to be Ct, and results were expressed as delta Ct (ΔCt) by subtracting the Ct values of the un-stimulated samples (containing only a PBS or DMSO vehicle) from those of the stimulated samples. Since Ct is a log scale, 1 ΔCt generally means double or one half in quantity, and negative ΔCt means an increase in expression. The Ct was determined by analytical software (SDS, ABI). In order to validate the assay, spiked RNA was also quantitated. Although 1x RT buffer was used as negative controls, none of the primer pairs produced non-specific primer-dimers under these experimental conditions. For PUMA in Fig. 8, immobilized cDNA on the oligo(dT)-immobilized microplates was directly amplified in an iCycler (BioRad).

The results are shown in Figure 5. In the Figure, heparinized whole blood was incubated at 37°C for 0-8 hours after stimulation of 30 Gy radiation (●), 20 µM BLM (▲), 100 µM VP-16 (◆), or corresponding controls ○ (no radiation), Δ (PBS), ◊ (DMSO). The RNA levels of control RNA34 (A), p21 (B), and PUMA (C) were quantitated as described above and delta Ct (ΔCt, Y-axis) was calculated. Each data point was the mean ± standard deviation derived from triplicate aliquots of whole blood. As shown in Figure 5A, ΔCt derived from spiked RNA (RNA34) were all within ±1 among the various stimulations and controls. This validated that the assay was reproducible and reliable. The p21 was used to confirm experimental conditions, as it is known to be induced via activation of the transcription factor p53 during DNA damage. The p21 mRNA increased without any stimulation. However, the levels of p21 were all significantly increased after these 3 stimulations (Fig. 5B). The p21 levels increased continuously for 8 hours when blood was stimulated with radiation or BLM, whereas VP-16 exhibited transient induction with a peak around 2-4 hours (Fig. 5B). Since the goal was to identify early gene markers, incubation was fixed at 4 hours at 37°C. The cDNA was then used to screen various pro-apoptotic mRNAs by SYBR green real time PCR. The results of this screening of various pro-apoptotic mRNAs are shown in Figure 6. In the Figure, heparinized whole blood was stimulated with or without 30 Gy radiation (A), 20 µM BLM (B), or 100 µM VP-16 (C) for 4 hours at 37°C, then various mRNA were quantitated. Each bar was the mean ± standard deviation of ΔCt from triplicate aliquots of each person's whole blood (5 for radiation (3 for Bcl-2 and Bcl-w), 3 for BLM and VP-16). As shown in Fig. 6, all of the tested individuals (5 for radiation (3 for Bcl-2 and Bcl-w), 3 each for BLM and VP-16) showed significant p21 induction, with ΔCt more than 2. Bax was also induced; however, the ΔCt was smaller than that of p21 (Fig. 6). Moreover, Bax was not a completely reliable marker, as it was not induced in 1 or 2 individuals, although it may be employed in the methods disclosed herein. PUMA was the most dominant mRNA, with results similar to those of p21, and responses were confirmed in all individuals (Fig. 6). NOXA was also induced in all individuals, but the degree of induction was less than that of PUMA (Fig. 6). Accordingly, among markers measured to indicate apoptosis, and thus drug effect, in the present method, PUMA, NOXA, and BAX may all be employed, with PUMA the most preferred mRNA. Although these mRNAs were employed, the present invention also contemplates the use of the other members of the Bcl-2/Bax family and BH3-only Bcl-2 family members described above. Furthermore, the use of other mRNAs that indicate susceptibility of cancerous cells to pharmaceutical agents is also contemplated. For example, the mRNA of the ATP-binding cassette (ABC) subfamilies A-G may be used. These genes encode membrane transporter proteins involved in trafficking of biological molecules across membranes and host-defense mechanism to xenobiotics. Specifically, the genes include ABC1, ABCA2 variant 1, ABCA2 variant 2, ABCA3, ABCA4, ABCA5 variant 1, ABCA5 variant 2, ABCA6 variant 1, ABCA6 variant 2, ABCA7 variant 1, ABCA7 variant 2, ABCA8, ABCA9 variant 1, ABCA9 variant 2, ABCA10, ABCA12 variant 1, ABCA12 variant 2, ABCA13, ABCB1, TAP1, TAP2 variant 1, TAP2 variant 2, ABCB4 variant A, ABCB4 variant B, ABCB4 variant C, ABCB5, ABCB6, ABCB7, ABCB8, ABCB9 variant 1, ABCB9 variant 2, ABCB9 variant 3, ABCB9 variant 4, ABCB10, ABCB11, ABCC1 variant 1, ABCC1 variant 2, ABCC1 variant 3, ABCC1 variant 4, ABCC1 variant 5, ABCC1 variant 6, ABCC1 variant 7, ABCC2, ABCC3 variant MRP3, ABCC3 variant MRP3A, ABCC3 variant MRP3B, ABCC4, ABCC5 variant 1, ABCC5 variant 2, ABCC6, ABCC8, ABCC9 variant SUR2A, ABCC9 variant SUR2B, ABCC9 variant SUR2A-delta-14, ABCC10, ABCC11 variant 1, ABCC11 variant 2, ABCC11 variant 3, ABCC12 variant A, ABCC12 variant B, ABCC12 variant C, ABCC12 variant D, ABCC12 variant E, ABCC13 variant 1, ABCC13 variant 2, ABCC13 variant 3, ABCC13 variant 4, ABCD1, ABCD2, ABCD3, ABCD4 variant 1, ABCD4 variant 2, ABCD4 variant 3, ABCD4 variant 4, ABCD4 variant 5, ABCF1 variant 1, ABCF1 variant 2, ABCF2 variant 1, ABCF2 variant 2, ABCF3, ABCE1, ABCG1 variant 1, ABCG1 variant 2, ABCG1 variant 3, ABCG1 variant 4, ABCG1 variant 5, ABCG1 variant 6, ABCG1 variant 7, ABCG2, ABCG4, ABCG5, and ABCG8. Among these, particularly preferred mRNAs are those for the ABCC2 gene product, the substrates for which include vinblastine, and the ABCG2 gene product, which has been identified as a candidate protein responsible for cancer multidrug resistance.

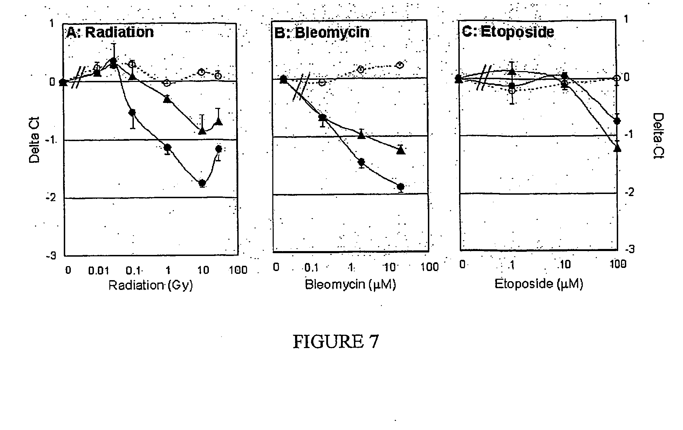

In order to confirm the results of Fig. 6, PUMA was further characterized by TaqMan real time PCR under the same conditions as shown in Fig. 5. Unlike p21, base line PUMA expression was unchanged during 8 hours' incubation at 37°C (Fig. 5C). Upon stimulation, PUMA was induced rapidly, with similar kinetics and degrees to those of p21 (Fig. 5C). In Fig. 7, dose dependency was also analyzed for RNA34, p21 and PUMA. The ΔCt of the control RNA34 was unchanged (Fig. 7). In Figure 7, heparinized whole blood was incubated at 37°C for 4 hours after stimulation with 0-30 Gy radiation (A), 0-20 µM BLM (B), or 0-100 µM VP-16 (C), then control RNA34 (○), p21 (▲), and PUMA (●) were quantitated. Each data point was the mean ± standard deviation derived from triplicate aliquots of whole blood. Significant induction of p21 and PUMA was observed at more than 0.1 (PUMA) -1 (p21) Gy of radiation, 0.1 µM BLM, and 100 µM VP-16, respectively (Fig. 7).

Detection of a drug-induced early apoptosis signal can be applicable to drug sensitivity tests for leukemia and lymphoma, where the majority of circulating leukocytes are leukemic. In order to determine whether p21 and PUMA were good apoptosis markers in abnormal leukocytes as well as in normal leukocytes in healthy individuals (Figs. 5-7), clinical studies were undertaken. Fig. 8 shows 3 clinical examples: chronic lymphocytic leukemia (A: CLL), acute myeloid leukemia (B: AML), and leukemic non-Hodgkin's lymphoma (C: NHL). More than 70% of the peripheral blood leukocytes were atypical. Heparinized whole blood was incubated with various drugs for 2 hours at 37°C, and RNA34, p21, Bax, and PUMA were quantitated. Specifically, heparinized whole blood from patients with CLL (A), AML (B) and NHL (C) were incubated with various anti-cancer drugs, and incubated at 37°C for 2 hours. Then RNA34 (□), p21 (■) and PUMA ( 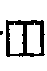 ) mRNA was quantitated as described above. Each bar was the mean ± standard deviation of ΔCt from triplicate aliquots of whole blood. The drugs used were vinblastine (VLB, 1 µM in final concentration), vincristine (VCL, 0.5 µM), pirarubicin (THP, 0.5 µM), mitoxantrone (MIT, 0.1 µM), peplomycin (PEP, 0.5 µM) aclarubicin (ACR, 0.1 µM), bleomycin (BLM, 0.5 µM), daunorubicin (DNR, 2.5 µM), doxorubicin (DXR, 1 µM), etoposide (VP-16, 10 µM), carboplatin (CBDCA, 10 µM), cisplatin (CDDP, 10 µM), Fludarabine (FDB, 1 µM), methotrexate (MTX, 10 µM), 5-fluorouracil (5-FU, 10 µM), cytarabine (Ara-C, 10 µM), dacarbazine (DTIC, 10 µM), and cyclophosphamide (CPA, 1 µM). Since these drugs are administered intravenously in clinical practice, the whole blood experiments employed are a simulation of actual clinical conditions. The results of RNA34 (Fig. 8A-C) and BAX (data not shown) were all less than 1 ΔCt. As shown in Fig. 8A, PUMA was induced by MIT and DNR, whereas p21 was not induced by any of these drugs in this CLL patient. In Fig. 8B, significant p21 induction was only seen with VLB, whereas a huge induction of PUMA was confirmed with THP, MIT, PEP, VP-16, CDDP, FDB, and MTX. The NHL patient in Fig. 8C showed a strong PUMA response against THP, MIT, VP-16, and MTX, whereas p21 responses were all less than 2 ΔCt. These results suggested that PUMA was functionally responsive in abnormal leukocytes, and unlike normal leukocytes, it was more sensitive than p21 in some patients.

The present embodiment contemplates that blood from a patient suffering from, e.g., cancer is assayed to tailor drug selection in the treatment protocol to be employed. In the contemplated method, blood is withdrawn from the patient, and exposed to the specific anticancer agents contemplated for the treatment protocol at the contemplated dosages. Suitable anticancer agents include all drugs prescribed for the treatment of a cancer; the specific drugs contemplated include but are not limited to etoposide, bleomycin, vinblastine, vincristine, pirarubicin, mitoxantrone, peplomycin, aclarubicin, daunorubicin, doxorubicin, carboplatin, cisplatin, Fludarabine, methotrexate, 5-fluorouracil, cytarabine, dacarbazine, cyclophosphamide, and the agents discussed in Katzung, Basic & Clinical Pharmacology (8^{th} ed. 2001), pp. The mRNA levels of p21 and PUMA (or alternatively NOXA) are then measured. Measurement after exposure for seven hours or less is preferable. Measurement after exposure for four hours or less is more preferable. Measurement after exposure for two hours or less is still more preferable. Measurement after exposure for other periods is also contemplated. Suitable measurement methods include, for example, real-time PCR using the TaqMan or SYBR Green systems, or any other systems for the measurement of mRNA levels known to those of skill in the art. The measured levels are then compared to levels in the blood before exposure to the agents or, preferably, to control samples in which the patient's blood was exposed for an equivalent amount of time to a control vehicle. These control vehicles include solvents in which the drugs are dissolved for use in the present method, such as DMSO and PBS. Depending on the response of the individual patient's blood to the treatment protocol, the actual protocol employed can be adjusted by increasing or decreasing the dosage, or by altering the agents used. In the case of cancers of the blood, the results are used to maximize the chance of cure, because the method provides direct data as to the effectiveness of a treatment regimen on the circulating blood cells that are cancerous. In the case of a solid tumor, the results are used to assess the risk of side effects such as blood leukocyte suppression that accompanies a particular protocol, so as to maximize the effect of the treatment on the tumor while minimizing toxicity.

### Embodiment 3: Assessment of Leukocyte Suppression

Blood samples were obtained from healthy volunteers or patients with leukemia and lymphoma. Triplicate aliquots of 50 µL each of heparinized whole blood was incubated with various concentrations of anti-cancer drugs (BLM, VP-16, and taxol) tor a specified length of time, then mRNA was purified, cDNA was synthesized, and the levels of p21, PUMA, and BAX were quantitated by TaqMan real time polymerase chain reaction (PCR), as described above. In brief, each blood sample was applied to 96-well filterplates to trap leukocytes. Lysis buffer containing artificial RNA (RNA34) and a cocktail of specific primers were added to filterplates, and cell lysates were transferred to oligo(dT)-immobilized microplates (GenePlate, RNAture, Irvine, CA) for hybridization. The DNA was then synthesized in the oligo(dT)-immobilized microplates without additional primers, and was used for TaqMan real time PCR in 384-well plates (Applied Biosystems, Foster City, CA). In order to validate each assay condition, spiked RNA was also quantitated. The sequences of primers and probes were designed by Primer Express (Applied Biosystems) and HYBsimulator (RNAture). The sequences used are shown in Table 2 below.

**TABLE 2: PRIMER AND PROBE SEQUENCES**

| Target | Sequences (5'-3') |
|---|---|
| RNA34 | |
| TaqMan probe* | CCAAGGCCCA GCCCTCACAC A (SEQ ID NO: 41) |
| forward primer | AGCCCCCTCACTCCCAAA (SEQ ID NO : 42) |
| reverse primer | GGGTGCTGTG CTTCTGTGAA C (SEQ ID NO: 43) |

| P21 | |
|---|---|
| TaqMan probe* | CCACTCCAAA CGCCGGCTGA TC (SEQ ID NO: 44) |
| forward primer | TTCTGCTGTC TCTCCTCAGA TTTCT (SEQ ID NO: 45) |
| reverse primer | GGATTAGGGCTTCCTCTTGGA (SEQ ID NO: 46) |

| PUMA | |
|---|---|
| TaqMan probe* | CCCCGCCCCA TCAATCCCA (SEQ ID NO: 47) |
| forward primer | GGGCCCAGACTGTGAATCCT (SEQ ID NO: 48) |
| reverse primer | ACGTGCTCTCTCTAAACCTATGCA (SEQ ID NO: 49) |

| BAX | |
|---|---|
| TaqMan probe* | TTGTCGCCCT TTTCTACTTT GCCAGCA (SEQ I D NO: 50) |
| forward primer | TTTCTGACGG CAACTTCAAC TG (SEQ ID NO: 51) |
| reverse primer | GGTGCACAGG GCCTTGAG (SEQ I D NO: 52) |

| | |
|---|---|
| *: 5'-FAM, 3'-TAMRA | |

Oligonucleotides were synthesized by IDT (Coralville, IA). The PCR cycle where certain amounts of PCR products were generated was determined to be Ct, and delta Ct (ΔCt) was also calculated by subtracting Ct values of the un-stimulated samples (exposed only to a control vehicle such as DMSO or PBS) from those of the stimulated samples. Since Ct is a log scale, 1 ΔCt generally means double or one half in quantity, and negative ΔCt means an increase in expression. For accurate statistical analysis (Student's t-test), triplicate aliquots of whole blood were used as starting materials. Since p21 is responsive for cell cycle arrest, and PUMA belongs to the pro-apoptotic BH3 domain only gene family, analysis of these genes corresponds to the cytostatic and cytocidal effects of each drug. Among many pro-apoptotic genes within BAX and BH3 domain only gene families, PUMA was measured, in accordance with the results disclosed in Embodiment 2 above. BAX was also measured in some cases.

Genomic DNA was purified from 300 µl each of whole blood with or without treatments, using a commercial kit (Puregene, Gentra, Minneapolis, MN). DNA was then analyzed in 3.5% agarose (3:1 NuSieve:agarose, FMC, Rockland, ME) gel electrophoresis, stained with 0.5 µg/ml ethidium bromide, and photographic images were recorded by Alpha-Iimager (Alpha Innotech, San Leandro, CA).

Fragmentation of DNA, a typical sign of apoptosis, was confirmed when whole blood was stimulated with VP-16 or BLM for 1 day (Fig. 5A, inset). As shown in Figure 5A, ΔCt derived from spiked RNA were all within +1 among various stimulations and controls. The variation within triplicate aliquots of whole blood was also less than 1 ΔCt (Fig. 5A-C, error bars). These data validated that the assay was reproducible and reliable. The p21 mRNA increased without any stimulation. However, the levels of p21 were all significantly increased upon stimulation with VP-16 or BLM (Fig. 5B). The p21 and PUMA levels increased continuously for 8 hours when blood was stimulated with BLM, whereas VP-16 exhibited transient induction with the peak around 2-4 hours (Fig. 5B). According to the dose response curves of VP-16 and BLM on p21 and PUMA expression shown above, 10-100 µM VP-16 and 0.2-20 µM BLM were used in the subsequent studies.

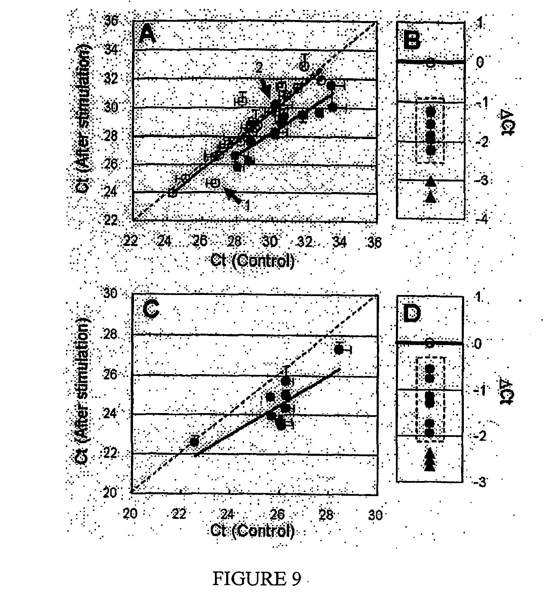

In Fig. 9A and C, the Ct values of p21 (A) or PUMA (C) of vehicle (DMSO)-treated blood sample (x-axis) were compared to those of VP-16 stimulated ones (Y-axis). In the Figure, triplicate aliquots of 50 µL each of heparinized whole blood was incubated with 10 (O) or 100 (●) µM VP 16 for 2 hours at 37°C. Then, levels of p21 (A, B) and PUMA (C, D) mRNA were measured as described above. In A and C, the Ct values of unstimulated (X-axis) and stimulated (Y-axis) samples were compared. Dotted lines indicate the line of equal values of both unstimulated and stimulated samples. Solid lines are regression lines for each dose. In B and D, delta Ct (ΔCt) was calculated by subtracting the Ct values of unstimulated samples from those of the stimulated ones. In the Figure, O indicates a non-responder, ● indicates moderate responders (n=9 for B, n=6 for D), and ▲ indicates high responders. Each data represents the mean±standard deviation from triplicate aliquots of whole blood in each individual. Arrow 1 indicates an individual who showed p21 induction at 10 µM of VP-16, and arrow 2 indicates another individual who showed no p21 induction at 100 µM of VP-16.

As shown in Fig. 9A, a low dose (10 µM) of VP-16 failed to induce p21 in many cases. However, 1 individual expressed significant p21 induction (Fig. 9A: arrow 1). The regression line was y = 1.0529x - 1.7719 (r² = 0.883). When the VP-16 dose was increased to 100 µM, the majority of individuals exhibited increased p21 expression. However, one individual showed no induction at all (Fig. 9A: arrow 2, 9B: open circle). The regression line was y = 0.7936x + 4.427 (r² =0.7905). VP-16 also induced PUMA at 100 µM (Fig. 9C) with regression line of y = 0.7529x + 4.9108 (r² =0.625). Interestingly, once these data were converted to ΔCt as shown in Fig. 9B and D, non-responders (open circle, n=1), moderate responders (closed circles: n=9 for p21 and n=6 for PUMA) and high responders (closed triangles: n=2 for p21 and n=3 for PUMA) were identified.

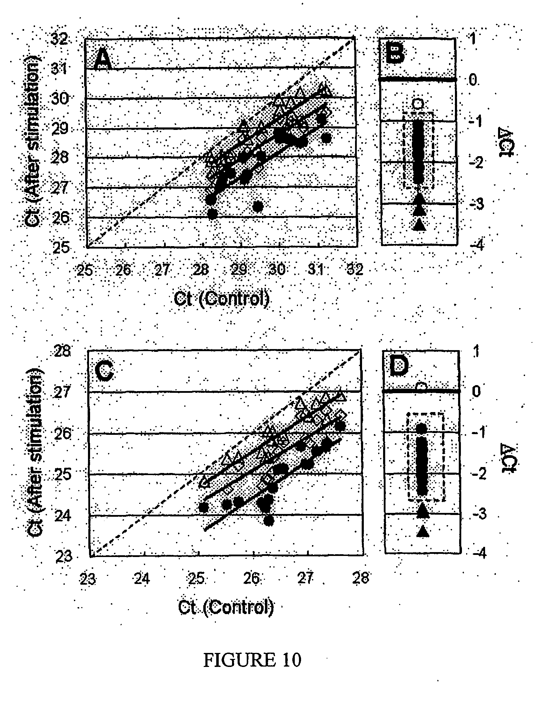

In contrast to VP-16, BLM induced both p21 and PUMA at concentrations as low as 0.2 µM in all cases (Fig. 10A, C). In the Figure, triplicate aliquots of 50 µL each of heparinized whole blood was incubated with 0.2 (Δ), 2 (◇) or 20 (●) µM BLM for 2 hours at 37°C. Then, the level of p21 (A, B) and PUMA (C, D) mRNA was determined as described above. In A and C, Ct values of unstimulated (X-axis) and stimulated (Y-axis) samples were compared. Dotted and solid lines are same as Fig. 9. In B and D, ΔCt was calculated by subtracting Ct values of the unstimulated samples from the stimulated samples. In the Figure, O indicates non-responders, ● indicates moderate responders (n=22 for B, n=20 for D), and ▲ indicates high responders. Each data represents the mean from triplicate aliquots of whole blood in each individual.

The regression lines of p21 for 0.2, 2, and 20 µM BLM were y = 0.8487x + 3.8275 (r² = 0.8367), y = 0.8082x + 4.513 (r² = 0.8444), and y = 0.8146x + 3.7147 (r² = 0.7552), respectively. PUMA responses were similar to those of p21, and the regression lines of PUMA for 0.2, 2, and 20 µM BLM were y = 0.8387x + 3.7409 (r² = 0.8654), y = 0.793x + 4.4688 (r² = 0.7254), and y = 0.8764x + 1.6353 (r² = 0.7546), respectively (Fig. 10C). When these data were converted to ΔCt, non-responders or low responders (open circle: n=1), moderate responders (closed circles: n=22 for p21 and n=20 for PUMA) and high responders (closed triangles: n=3) were identified (Fig. 10B and D).

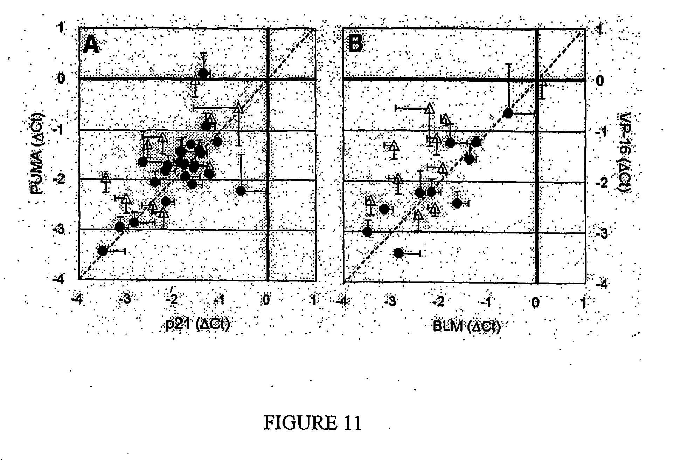

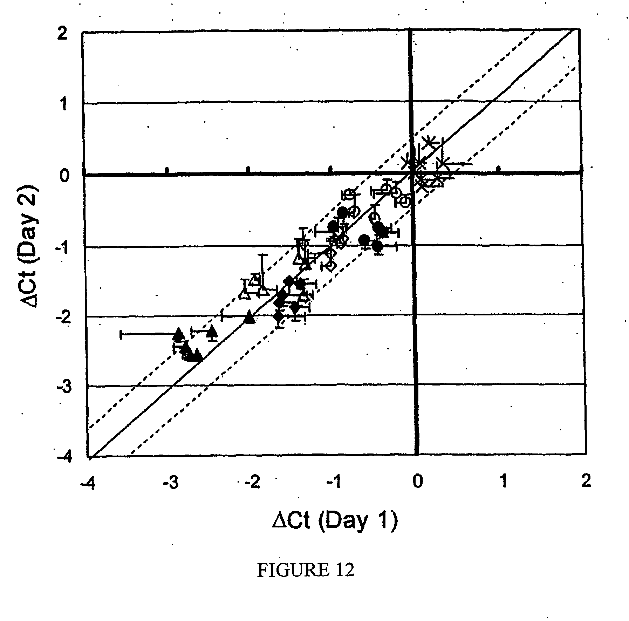

Fig. 11 summarizes cases in which p21 and PUMA were analyzed for both VP-16 and BLM stimulations. In the Figure, the data in Fig. 9-10 were re-plotted to compare p21 (●) and PUMA (Δ) responses (A), and VP-16 (Δ) and BLM (●) responses (B). Dotted lines are the same as in Fig. 9 and 10. Each data represents the mean±standard deviation from triplicate aliquots of whole blood in each individual. In Fig. 11A, high and low responders identified by p21 were also identified as similar responders by PUMA. Similarly, as shown in Fig. 11B, high and low responders against VP-16 were also identified as high and low responders against BLM. In order to analyze day-to-day variation of drug responses, the experiments were repeated twice by drawing blood samples from the same individuals within 1-3 days. The results are shown in Figure 12. Symbols are the mean±standard deviation from each individual. In the Figure, ● indicates 2 µM BLM-induced p21, O indicates 2 µM BLM-induced PUMA, ◆ indicates 20 µM BLM-induced p21, ◇ indicates 20 µM BLM-induced PUMA, ▲ indicates 100 µM VP-16-induced p21, Δ indicates 100 µM VP-16-induced PUMA, and X indicates control unstimulated RNA34, respectively. The solid line is the line where two data are identical. Dotted lines are ±0.5 ΔCt. As shown in Fig. 12, the VP-16- and BLM-induced induction of p21 and PUMA was comparable between the 2 samples, and variation was within +0.5 ΔCt.

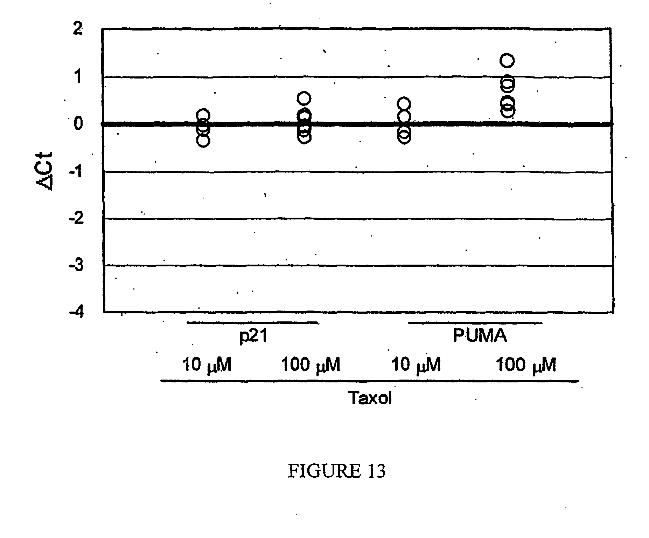

The same methodology was applied to a different class of anti-cancer drug, taxol. Taxol is a microtubule poison, and its induction of apoptosis has been shown to increase the expression of pro-apoptotic BAK and BAX. However, the effect of taxol on blood cells is very weak, and IC₅₀ is larger than 10 mM. In the present study, taxol also failed to induce both p21 and PUMA mRNA in all individuals even at concentration as high as 100 µM (Fig. 13). In the Figure, triplicate aliquots of 50 µL each of heparinized whole blood was incubated with 10 or 100 µM taxol for 2 hours at 37°C. Then, p21 and PUMA were determined and ΔCt was calculated as described above. Each data represents the mean from triplicate aliquots of whole blood in each individual. Taxol was not used at concentrations higher than 100 µM, because higher than 100 µM is beyond blood levels.

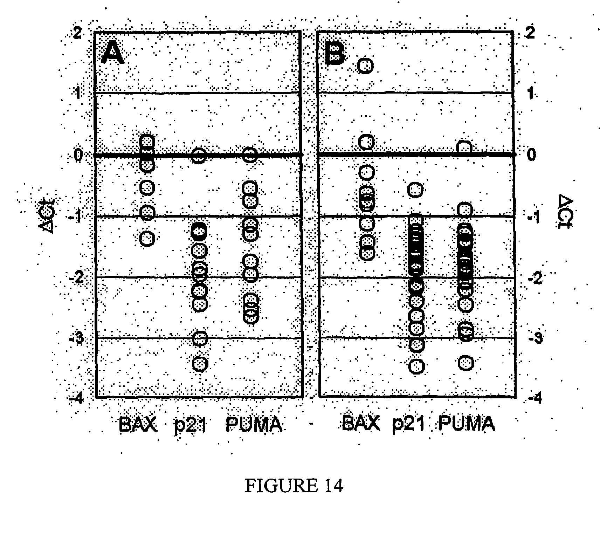

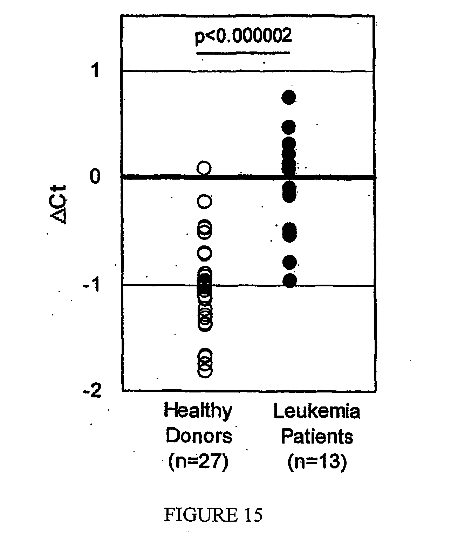

Figure 14 shows a comparison of BAX expression to that of p21 and PUMA. In the Figure, triplicate aliquots of 50 µL each of heparinized whole blood was incubated with or without 100 µM VP-16 (A) or 20 µM BLM (B) for 2 hours at 37°C. Then, BAX, p21, and PUMA mRNA was quantitated and ΔCt was calculated as described above. Each data point was the mean from triplicate determinations. BAX responses to VP-16 and BLM were significantly less than those of p21 and PUMA (p<0.01). In Fig. 15, BLM-induced p21 responses in healthy donors were compared to those of leukemia and lymphoma patients, in which more than 80% of leukocytes were leukemic cells. In the Figure, triplicate aliquots of 50 µL each of heparinized whole blood was incubated with or without 1 µM BLM for 2 hours at 37°C. Then, p21 mRNA was quantitated as described above. ΔCt values derived from 27 healthy donors' blood samples were compared with those of 13 patients with leukemia and lymphoma, in which more than 80% of leukocytes were cancerous cells. Each data point is the mean from triplicate determinations. Interestingly, BLM responses in leukemia and lymphoma population were significantly (p=0.000002) less than those of healthy control, suggesting that these cancerous cells were resistant to BLM. This confirmed the fact that BLM was not an appropriate choice of drug for these diseases.

The present ex vivo gene expression analysis is sensitive enough to detect drug-induced gene expression of apoptosis-related mRNAs from as small as 70 µL of whole blood (actual volume of 50 µL plus 20 µL reserve), which allows the analysis of 28 data sets in triplicate from 1 blood tube (6 mL) (6000/70/3=28.6). This is more than enough for analyzing 3 drugs (VP-16, BLM, and taxol) with 3 doses (0.2-100 µM) plus unstimulated controls (DMSO for VP-16, PBS for BLM and taxol), requiring only 11 data sets. Unlike serum experiments, where serum volume recovered from 6 mL of blood varies among individuals, whole blood experiments permit the design of a detailed analysis from a fixed volume of blood. Conventional assays use isolated mononuclear cells, suspending them in artificial cultured media. Although these assays are an excellent research model for the analysis of the cell itself, they ignore cell-to-cell and cell-to-plasma interaction, unavoidable considerations for the understanding of human physiological conditions as a whole. As ACD and EDTA chelate calcium, a critical component for many biological activities, heparin is the preferred anticoagulant.

Since mRNA transcription is an upstream event of protein synthesis and happens within a couple of hours after stimulation, mRNA analysis occurs in conditions closer to physiological conditions than subsequent protein analysis or measurement of biological activities, where a much longer incubation is required. The longer the incubation, the more secondary effects may happen. This is particularly important when individual-to-individual variation is considered. mRNA analysis also has the advantage of using gene amplification technologies, which makes this assay more sensitive than protein analysis or cell-based functional assays, and 50 µL of whole blood is enough for the analysis of at least 20 genes.

When up- or down-regulation of gene expression is analyzed, the assay sensitivity is dependent on the variation of each data set, although gene amplification technologies are capable of detecting a single copy of the target gene in test tubes. For example, if the coefficient of variation (CV) of 2 data sets is 10% in each case, approximately 20% of changes in gene expression can be detected with statistical significance. If the CV becomes 30%, more than 70% changes are required for statistical significance. Ex vivo whole blood assays involve a number of factors which increase CV. For example, pipetting of viscous whole blood, RNA isolation step, cDNA synthesis reaction, and PCR all induce variation. Since PCR amplifies the target gene exponentially, a small variation in any step becomes unacceptably wide at the end. Moreover, variation of ΔCt becomes much larger because it is the multiplex sum of two assays. The present method demonstrates an extremely small variation even when triplicate whole blood aliquots are used as starting materials, and ΔCt is calculated. As a result, it is possible to identify minute changes in drug-induced up-regulation of p21, PUMA, and BAX.

If certain drugs (such as VP-16 and BLM) (Fig. 13-14) induce p21 and PUMA under physiological conditions ex vivo, such drugs are expected to be more likely to induce leukocyte problems than those of negative ones (such as taxol) (Fig. 13) in each individual. Notably, one individual failed to induce p21 and PUMA even VP-16 concentration was as high as 100 µM (Fig. 9). Such an individual would have a low risk of leukocyte suppression if that drug were employed. Cancerous cells are also less responsive to BLM (Fig. 15). Identification of these non-responders or high-responders will enable the analysis of drug responses and resistance in humans, which can not be replaced by experiments with animals or cultured cells.

### Embodiment 4: Clinical Results in Cancer Treatment

In order to show that the method for the tailored administration of drugs of the present invention resulted in clinically useful results, the methods described above were employed in a study of nine patients suffering from various forms of lymphoma and leukemia. The patient population is shown in Table 3 below. In the Table, "FL" indicates follicular lymphoma, "MCL" indicates mantle cell lymphoma, "AML" is acute myeloid leukemia (M2 indicates the stage), "T-" indicates T-cell type, and "ALL" indicates acute lymphoblastic leukemia.

**TABLE 3: CLINICAL PATIENT POPULATION**

| | **Age/Sex Diagnosis** | | **Previous Rx** | **Chromosomal anomaly/Surface marker** |
|---|---|---|---|---|
| 1 | 60F | FL | (-) | 46XX: |
| 2 | 66F | MCL | (-) | CD 5-,10+, 19+, 20+, 23+, DR+ CD 5+,10-, 19+, 20+, cyclinDI+ |
| 3 | 71 M | T-ALL | (-) | |
| 4 | 41M | AML(M2) | (-) | t(8,21), t(10,20), del(9)(q21q31 q31): CD 13+, 33+, 34+, D R+ |
| 5 | 39F | t-AML | AraC,DNR,MIT,VP-16 IDR,ACR,I-PAM-resistant | Chromosome 5 and 7 abnormality CD 13+, 33+, 34+, DR+ |
| 6 | 45F | AML(M2) | (-) | del 11q21, t(1 20)(q34;p13); CD 13+, 33+, 34+, D R+ |
| 7 | 64M | AML(M2) | (-) | add(7)(q22), +8, +9: CD 13+, 33+, 34+, DR+ |
| 8 | 57F | AML(M2) | (-) | 46XX: CD 13+, 33+, 34+, 7+, 19+, 56+, DR+ |
| 9 | 75F | AML(M2) | (-) | t(3;8): CD 13+. 33+, 34+,19+, 5R+, DR+ |

The whole blood of the patients was exposed to the drugs listed in Table 4 below, and the levels of p21, BAX, and in some cases PUMA in the patient's leukocytes were measured as described above after exposure for a period of four hours. Control blood samples were exposed only to the control vehicle solvent (DMSO or PBS, as shown in Table 4), and Ct and ΔCt were obtained from the measured mRNA values as described above.

**TABLE 4: ANTI-CANCER DRUGS USED**

| | **Drugs** | **Final dose** | **Solvent** |
|---|---|---|---|
| CPA | Cyclophosphamide | 10 µM | PBS |
| NIM | Nimustine | 10 µM | PBS |
| AraC | Cytarabine | 10 µM | PBS |
| 5-FU | Fluorouracil | 10 µM | PBS |
| DXR | Doxorubicin | 1 µM | PBS |
| DNR | Daunorubicin | 10 µM | PBS |
| BLM | Bleomycin | 1 µM | PBS |
| ACR | Aclarubicin | 0.1 µM | PBS |
| PEP | Peplomycin | 1 µM | PBS |
| VCR | Vincristine | 1 µM | PBS |
| VBL | Vinblastine | 1 µM | PBS |
| CBDCA | Carboplatin | 10 µM | PBS |
| CDDP | Cisplatin | 10 µM | PBS |
| MIT | mitoxantrone | 0.1 µM | PBS |
| DTIC | Dacarbazine | 10 µM | DMSO |
| L-PAM | Melphalan | 10 µM | DMSO |
| Flu | Fludarabine | 1 µM | DMSO |
| MTX | Methotrexate | 10 µM | DMSO |
| IDR | Idarubicin | 10 /1M | DMSO |
| THP | Therarubicin | 1 µM | DMSO |
| VP-16 | Etoposide | 10 µM | DMSO |

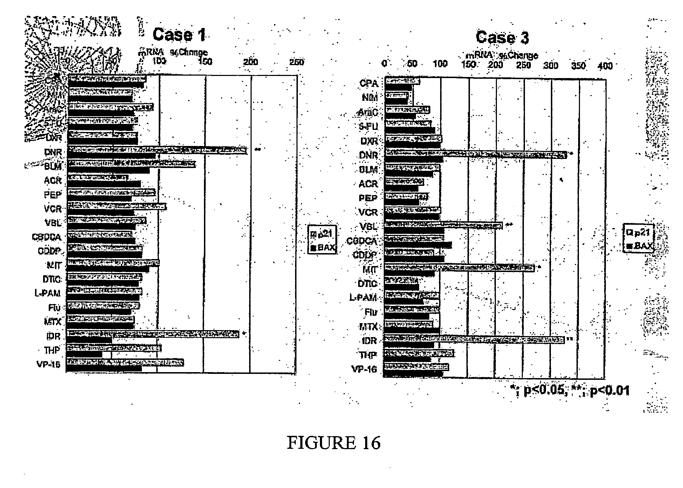

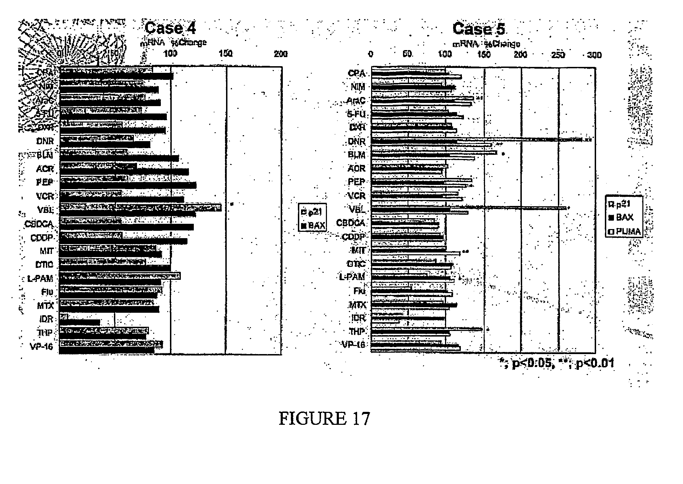

The results are shown in Figures 16 and 17, and in Table 5 below. As can be seen from the Figures, the drugs showing an increase in p21 or PUMA mRNA varied considerably from patient to patient. Although in this relatively small patient population BAX was in general not induced, the present method still contemplates the measurement of BAX and the other members of the Bcl-2/BAX family as possible markers for anticancer drug activity. As shown in the Figures, both p21 and PUMA showed an increase in mRNA levels as a result of stimulation with various drugs in selected patients.

**TABLE 5: CLINICAL RESULTS**

| **WBC/ µ l(%bl)** | | **Treatment** | **Inclusion of sensitive drugs** | **Clinical outcome** | **Judgment mRNA/Clinical*** |
|---|---|---|---|---|---|
| 1 | 200(92%) | CHOP | none | C | -/- |
| 2 | 3,900(52%a) | R-COP | none | C | -/- |
| | | R-THP-CVPE | THP(p21)** | B | +/+ |
| 3 | 2,600(34%) | VP | VDS (p21) | B | +/+ |
| 4 | 25,800(76%) | CAG | none | C | -/- |
| 5 | 45,300(85%) | RI | MIT, VP-16 (p21) | A | +/+ |
| 6 | 5,400(46%) | RI | DNR (p21, PUMA) | A | +/+ |
| 7 | 163,300(54%) | RI | none | A | -/+ |
| 8 | 28,300(49%) | RI | AraC, DNR (p21,PUMA) VP-16 (p21) | B | +/+ |
| | | CAG | ACR (p21, PUMA) | A | +/+ |
| 9 | 120,000(78%) | CA | none | C | -/- |
| | | VA' | none | C | -/- |

Table 5 shows the results of follow-on therapy, using either drugs for which individualized efficacy was suggested by the results obtained by the method of the present invention ("sensitive drugs") or using other drugs. In the Table, CHOP indicates therapy with CPA, Adriacin (ADR: doxorubicin), VCR, and predonisolone (PSL); R- indicates rituximab; COP indicates therapy with CPA, VCR, and PSL; VP indicates therapy with VDS and PSL; RI indicates therapy with AraC, DNR, MIT, and VP-16; CAG indicates therapy with low dose AraC, ACR, and G-CSF; CA indicates therapy with low dose AraC and ACR; and VA indicates therapy with low dose AraC and low dose VP-16. VDS indicates vindesine. In the "Judgment" column, A indicates complete remission, B indicates improved hematological test results, and C indicates no improvement.

Some patients were treated more than once. As shown in the Table, all of the seven courses of treatment with sensitive drugs resulted in an increase in marker mRNA and a favorable clinical outcome (shown as "+/+" in the Figure). Of these, three went into complete remission ("A" under "Clinical Outcome"). Two of the patients benefiting from sensitive drug therapy failed to respond to standard drug therapy with non-sensitive drugs. Of the six courses of treatment with non-sensitive drugs, only one resulted in a favorable clinical outcome. The remainder showed no improvement.

The method of the present invention thus suggested drug protocols tailored to each patient that, when followed, resulted in improved clinical outcomes, including complete remission in half of the successfully treated cases. This strongly indicates that the method of the present invention will be useful in tailoring treatment protocols in cancer cases to the individual patient, based on the drug sensitivities of the patient's diseased leukocytes as reflected in the change in the level of marker mRNAs within those leukocytes.

The results also show a close correlation between a positive mRNA result and a favorable clinical outcome: all but one outcome was +/+ or -/-. This correlation suggests that the method will also be useful for tailoring treatment protocols to avoid leukocyte suppression in cases in which a solid tumor is being treated with chemotherapy. If the leukocytes show a significant increase in the marker mRNA in response to a drug during this whole-blood assay, it is likely that leukocyte suppression will result from the growth arrest or apoptosis of the patient's leukocytes during therapy with that drug.

Embodiment 5: Tailored Drug Administration For Immunosuppression

Blood was obtained from healthy adults and from patients with psoriasis (4), aplastic anemia (1), nephrotic syndrome (1), and bone marrow transplantation (2). At the time of blood draw, one EDTA tube was sent to clinical laboratory for the measurement of leukocyte counts, and one heparin tube was stored on ice. Blood aliquots were stimulated with phytohemagglutinin-P (PHA-P) in the presence and absence of CSA at 5/°C for a vanous length of time. Fifty µL each of blood sample was applied to three different wells of filterplates (triplicate from the beginning), and leukocytes were collected on the membrane by centrifugation at 800 xg for 2 min. After washing each well with 300 µL phosphate buffered saline (PBS) once by centrifugation, the lysis buffer described above was applied to each well and incubated at 37°C for 10 min to release mRNA from trapped leukocytes. The lysate was then transferred to oligo-(dT)-immobilized microplates (GenePlate, RNAture, Irvine, CA) by centrifugation at 2000 xg for 5 min, and incubated at 4°C overnight for hybridization. After washing each well with 100 µL lysis buffer 3 times followed by 150 µL wash buffer (10 mM Tris, pH 7.4, 1 mM EDTA, pH 8.0, and 0.5 M NaCl) 3 times, cDNA was synthesized in each well by adding buffer, nucleotides, a cocktail of specific primers, RNasin, and MMLV reverse transcriptase (Promega, Madison, WI), and incubated at 37°C for 2 hours. The resultant cDNA (4 µL) was used for TaqMan real time PCR in a final volume of 10 µL in 384-well microplates using the thermal cycler (ABI, PRISM 7900).

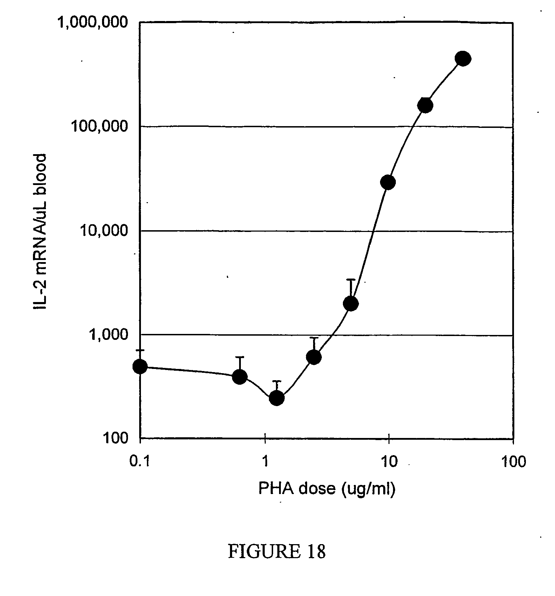

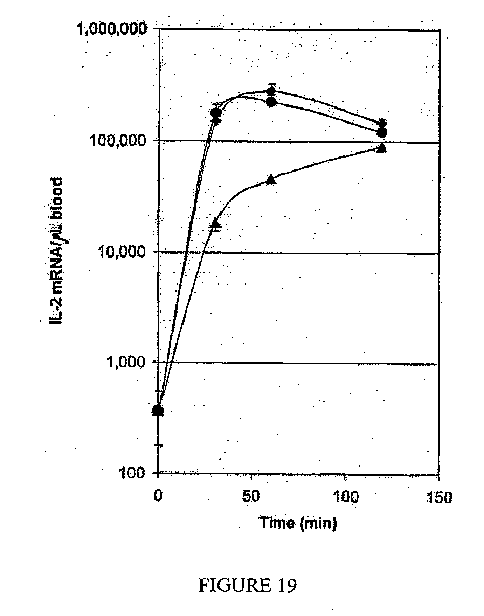

Fig. 18 shows the results of dose dependent induction of IL-2 mRNA by PHA-P in healthy adults. As shown in Fig. 18, IL-2 mRNA increased from 5 µg/mL PHA-P, and reached a plateau over 40 µg/mL PHA-P (Fig. 19). In Figure 19, triangles indicate a CsA concentration of 10 µg/mL, while diamonds indicate a concentration of 40 µg/mL, and circles indicate a concentration of 100 µg/mL. 100 µg/mL PHA-P was employed in the subsequent analysis in order to minimize individual variations. PHA-P was used for the induction of lymphocyte blastogenesis over a long period of time, where the action of PHA-P was maximal at 10-20 µg/mL, and the activity declined at higher doses. The IL-2 mRNA data were different, and the activity remained high even when 100 µg/mL PHA-P was used (Fig. 19). Other lectins, such as pokeweed mitogen, may also be employed. Unlike a lymphocyte blastogenesis assay, which takes 3-5 days in cultured media, this mRNA assay takes only 1-2 hours in whole blood (Fig. 19). The short incubation in whole blood mimics physiological conditions, and reduces assay variation substantially.

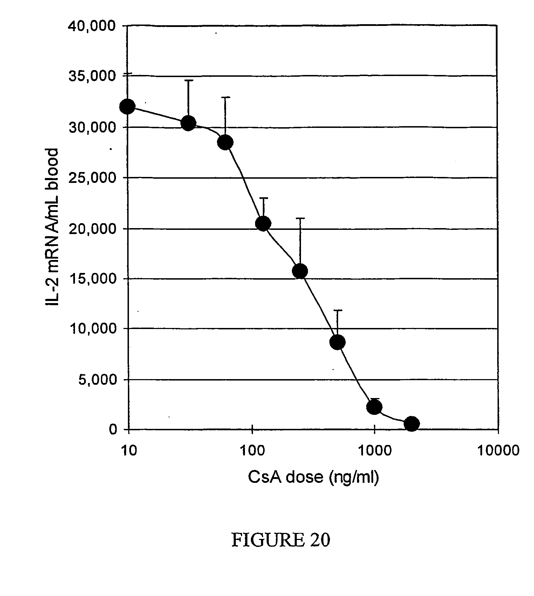

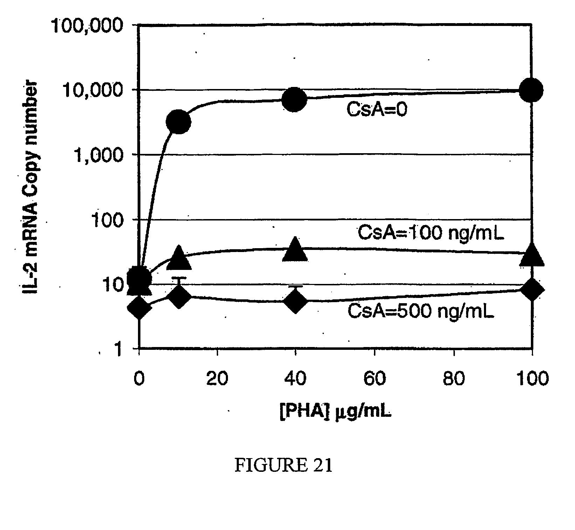

Various concentrations of CsA were incubated with heparinized whole blood of healthy volunteers for 30 min at 37°C, then IL-2 mRNA was induced by 10 µg/mL PHA-P at 37°C for 2 hours. As shown in Fig. 20, the transcription of IL-2 mRNA was inhibited by CsA in a dose dependent manner from 50-2000 ng/mL. By 2000 ng/mL CsA, IL-2 mRNA expression was inhibited almost 100% (Fig. 20). In Fig. 3, CsA was added into blood samples 30 min prior to the addition of PHA-P. However, similar inhibition was identified when CsA and PHA-P were added simultaneously (data not shown). Moreover, the inhibitory actions of CsA were maintained even when 100 µg/mL PHA-P was used (Fig. 21). In order to minimize assay variations, we used 100 µg/mL PHA-P for subsequent experiments.

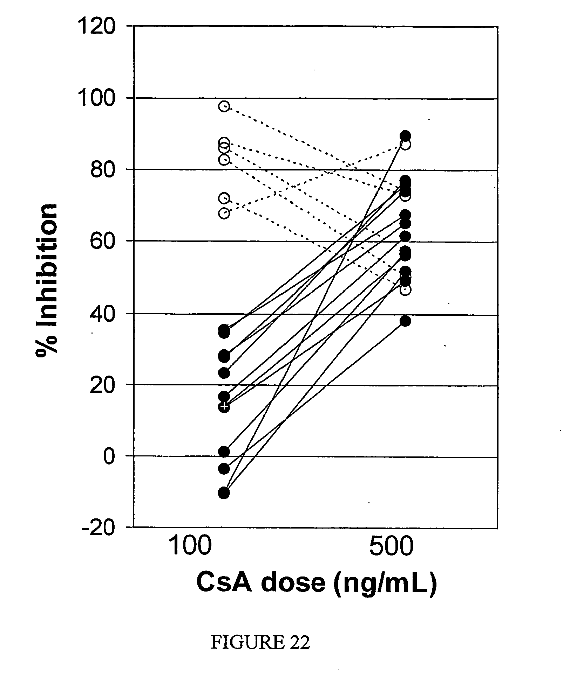

The experiments shown in Fig. 19 were repeated for 15 healthy donors during a one month period, with 1-5 blood samples in each experiment. The concentrations of CsA were 0, 100, and 500 ng/mL in each sample. As shown in Fig. 22, all 15 healthy donors exhibited significant inhibition of more than 40%. Interestingly, at 100 ng/mL CsA, healthy donors were divided into two groups: approximately 1/3 high responders (open circles), and 2/3 low responders (closed circles). This indicates clinical utility: low responders may be given higher doses of CsA, or switched to FK, and high responders may be given lower doses of CsA to reduce the chance of any adverse effect.

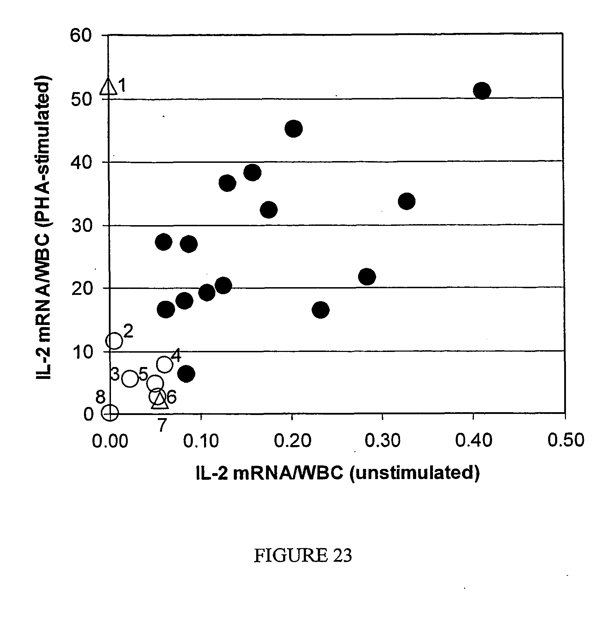

Next, a comparison was made between healthy adults and patients who were taking CsA or FK. The results are shown in Figure 23. In the Figure, the values of IL-2 mRNA were divided by the number of leukocytes in each sample, because leukocyte counts varied widely among patients. In the Figure, closed circles indicate healthy adults, open circles indicate CsA patients, and open triangles indicate FK patients. The patients had the following illnesses: 1: leukemia with bone marrow transplantation, 2: psoriasis, 3: aplastic anemia, 4: psoriasis, 5: psoriasis, 6: psoriasis, 7: leukemia with bone marrow transplantation, and 8: nephrotic syndrome. The x-axis is the baseline values of IL-2 mRNA without PHA-P stimulation, and the y-axis is the values after PHA-P stimulations. Closed circles were data from healthy adults, and open circles and triangles were derived from either CsA or FK patients. As shown in Fig. 23, patients with low baseline IL-2 mRNA showed small induction. Thus, there is no significant difference between these groups when % changes were calculated. However, once data were transformed to a 2D graph as shown in Fig. 6, plots of the patients were clearly distinguishable from those of healthy adults. Baseline IL-2 mRNA was 0.16±0.10 and 0.03±0.03 for healthy adults and patients, respectively, with statistical significance (p=0.001). IL-2 mRNA after PHA-P stimulation was 26.68±12.03 and 10.86±16.97 for healthy adults and patients, respectively, with statistical significance (p=0.007). Patient case number 1 showed a low baseline IL-2 mRNA with a high PHA-P response, indicating that medication was not effective in comparison with other patients. Patient case number 4 showed similar responses to those of the lower limit of healthy adults, also indicating poor drug responses. Although drug responses are the sum of complex biological activities in each patient, this functional responsiveness assay will provide clinicians with useful information for the management of their patients.

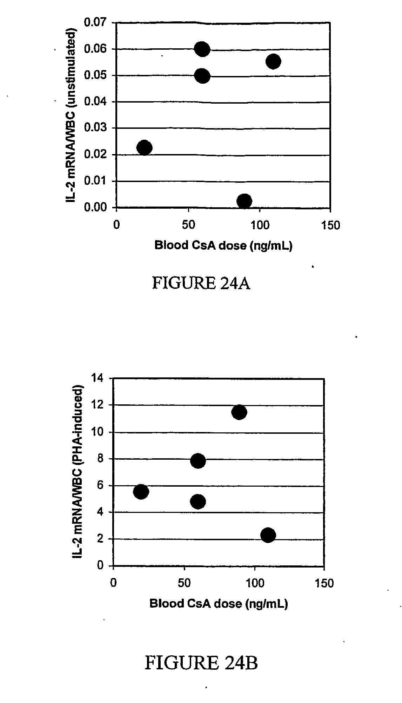

Fig. 24A and 24B show the relationship between baseline (Fig. 24A) or post PHA-P stimulated IL-2 mRNA (Fig. 24B) and blood levels of CsA. The mRNA values were divided by the number of leukocytes. As shown in the Figures, no clear relationship was identified. Since the blood levels of CsA in these patients were around 100 ng/mL, where high and low responders exist (Fig. 22), this functional assay has advantages over a simple measurement of drug concentration in that it can differentiate among these two populations.

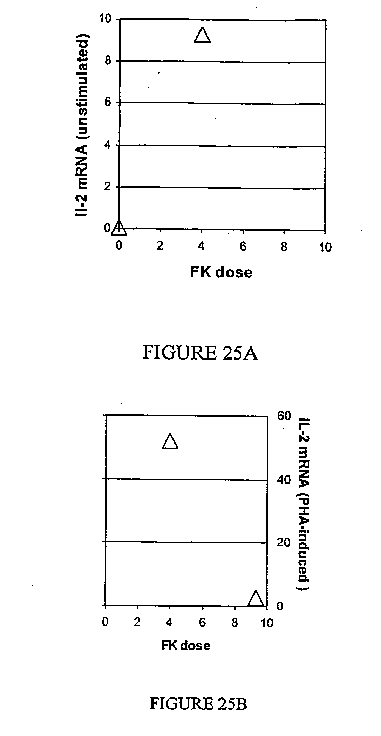

Fig. 25 shows the results when FK was employed. Although no clear conclusion was possible because of the small population size, it is expected that the method will be useful with FK as well.

## Claims

1. A method of measuring a blood cancer patient's responsiveness to an anti-cancer drug, comprising:
exposing heparinized whole blood of the patient to the drug ex vivo for 7 hours or less;
exposing heparinized whole blood of the patient to a control vehicle ex vivo for 7 hours or less;
after said exposure, measuring the amount of at least one mRNA selected from the group consisting of mRNAs encoding a PUMA gene product and a p21 gene product, both in the blood cells exposed to the drug and in the blood cells exposed to the control vehicle; and
identifying responsiveness to the drug by comparing results of the measurement obtained after exposure to the control vehicle with results of the measurement obtained after exposure to the drug, wherein a change in the amount of the mRNA indicates the patient's responsiveness to the drug.

2. The method of claim 1 wherein the control vehicle is selected from the group consisting of phosphate-buffered saline and dimethyl sulfoxide.

3. The method of claim 1, wherein the whole blood is stimulated for 5 hours or less.

4. The method of claim 1, wherein the whole blood is stimulated for 2 to 4 hours.

5. The method of claim 1, wherein the effect of the drug is apoptosis of blood cells.

6. The method of claim 1, wherein the effect of the drug is cell cycle arrest in blood cells.

7. The method of claim 1, additionally comprising measuring the amount of a second mRNA associated with a second effect of the drug in blood cells, and wherein
the first effect of the drug is apoptosis of blood cells and the first mRNA encodes the PUMA gene product; and
the second effect of the drug is cell cycle arrest in blood cells and the second mRNA encodes the p21 gene product.

8. The method of claim 5 or 6, wherein the drug is selected from the group consisting of etoposide, doxorubicin, fludarabine, mitoxantrone, rituximab, vindesine, pirarubicin, carboplatin, cyclophosphamide, bleomycin, vinblastine, vincristine, peplomycin, aclarubicin, daunorubicin, cisplatin, methotrexate, 5-fluorouracil, cytarabine, dacarbazine, cyclophosphamide, and paclitaxel.

9. The method of claims 5, 6, 7 or 8 wherein the patient suffers from leukemia or leukemic lymphoma.

10. The method of claim 9, wherein the lectin is selected from the group consisting of phytohemagglutanin-P and pokeweed mitogen.

11. A method of measuring a blood cancer patient's responsiveness to a drug selected from the group consisting of etoposide, doxorubicin, fludarabine, mitoxantrone, rituximab, vindesine, pirarubicin, carboplatin, cyclophosphamide, bleomycin, vinblastine, vincristine, peplomycin, aclarubicin, daunorubicin, cisplatin, methotrexate, 5-fluorouracil, cytarabine, dacarbazine, cyclophosphamide, and paclitaxel, comprising:
exposing heparinized whole blood of the patient to the drug ex vivo for 4 hours or less;
exposing heparinized whole blood of the patient to a control vehicle ex vivo for 4 hours or less;
after said exposure, measuring the amount of at least one mRNA selected from the group consisting of mRNAs encoding the p21 and PUMA gene products in blood cells;
comparing results of the measurement obtained after exposure to the control vehicle with results of the measurement obtained after exposure to the drug; and
identifying responsiveness of the drug based on the results of the comparison, wherein a change in the amount of the mRNA indicates the patient's responsiveness to the drug.

## Patentansprüche

1. Verfahren zur Messung des Ansprechens eines Blutkrebspatienten auf ein Krebsarzneimittel, umfassend:
mit Heparin versetztes Vollblut des Patienten wird unter ex-vivo-Bedingungen für eine Dauer von 7 Stunden oder weniger mit dem Arzneimittel in Kontakt gebracht,
mit Heparin versetztes Vollblut des Patienten wird unter ex-vivo-Bedingungen für eine Dauer von 7 Stunden oder weniger mit einem Kontrollvehikel in Kontakt gebracht,
nach dem Kontaktieren wird die Menge von mindestens einer mRNA gemessen, die aus der Gruppe ausgewählt ist, die aus mRNAs mit dem Kode für ein PUMA-Genprodukt und ein p21-Genprodukt besteht, und zwar sowohl in den mit dem Arzneimittel in Kontakt gebrachten Blutzellen als auch in den mit dem Kontrollvehikel in Kontakt gebrachten Blutzellen, und
Identifizieren des Ansprechens auf das Arzneimittel durch Vergleich der Ergebnisse der Messung, die nach dem Kontakt mit dem Kontrollvehikel erhalten wurden, mit den Ergebnissen der Messung, die nach dem Kontakt mit dem Arzneimittel erhalten wurden, wobei eine Änderung der mRNA-Menge das Ansprechen des Patienten auf das Arzneimittel anzeigt.

2. Verfahren nach Anspruch 1, wobei das Kontrollvehikel aus der Gruppe ausgewählt ist, die aus Phosphat-gepufferter Kochsalzlösung und Dimethylsulfoxid besteht.

3. Verfahren nach Anspruch 1, wobei das Vollblut für eine Dauer von 5 Stunden oder weniger stimuliert wird.

4. Verfahren nach Anspruch 1, wobei das Vollblut für eine Dauer von 2 bis 4 Stunden stimuliert wird.

5. Verfahren nach Anspruch 1, wobei es sich bei der Wirkung des Arzneimittels um die Apoptose von Blutzellen handelt.

6. Verfahren nach Anspruch 1, wobei es sich bei der Wirkung des Arzneimittels um die Zellzyklushemmung von Blutzellen handelt.

7. Verfahren nach Anspruch 1, zusätzlich umfassend die Messung der Menge einer zweiten mRNA, die mit einer zweiten Wirkung des Arzneimittels in Blutzellen in Verbindung steht, und wobei
es sich bei der ersten Wirkung des Arzneimittels um die Apoptose von Blutzellen handelt und die erste mRNA den Kode für das Genprodukt PUMA enthält und
es sich bei der zweiten Wirkung des Arzneimittels um die Zellzyklushemmung von Blutzellen handelt und die zweite mRNA den Kode für das Genprodukt p21 enthält.

8. Verfahren nach Anspruch 5 oder 6, wobei das Arzneimittel aus der Gruppe ausgewählt ist, die aus Etoposid, Doxorubicin, Fludarabin, Mitoxantron, Rituximab, Vindesin, Pirarubicin, Carboplatin, Cyclophosphamid, Bleomycin, Vinblastin, Vincristin, Peplomycin, Aclarubicin, Daunorubicin, Cisplatin, Methotrexat, 5-Fluoruracil, Cytarabin, Dacarbazin, Cyclophosphamid und Paclitaxel besteht.

9. Verfahren nach Anspruch 5, 6, 7 oder 8, wobei der Patient an Leukämie oder leukämischem Lymphom leidet.

10. Verfahren nach Anspruch 9, wobei das Lektin aus der Gruppe ausgewählt ist, die aus Phytohämagglutinin P und Mitogen aus Kermesbeerengewächsen ("pokeweed mitogen") besteht.

11. Verfahren zur Messung des Ansprechens eines Blutkrebspatienten auf ein Arzneimittel, das aus der Gruppe ausgewählt ist, die aus Etoposid, Doxorubicin, Fludarabin, Mitoxantron, Rituximab, Vindesin, Pirarubicin, Carboplatin, Cyclophosphamid, Bleomycin, Vinblastin, Vincristin, Peplomycin, Aclarubicin, Daunorubicin, Cisplatin, Methotrexat, 5-Fluoruracil, Cytarabin, Dacarbazin, Cyclophosphamid und Paclitaxel besteht, umfassend:
mit Heparin versetztes Vollblut des Patienten wird unter ex-vivo-Bedingungen für eine Dauer von 4 Stunden oder weniger mit dem Arzneimittel in Kontakt gebracht,
mit Heparin versetztes Vollblut des Patienten wird unter ex-vivo-Bedingungen für eine Dauer von 4 Stunden oder weniger mit einem Kontrollvehikel in Kontakt gebracht,
nach dem Kontaktieren wird die Menge von mindestens einer mRNA in Blutzellen gemessen, die aus der Gruppe ausgewählt ist, die aus mRNAs mit dem Kode für p21- und PUMA-Genprodukte besteht,
Vergleichen der Ergebnisse der Messung, die nach dem Kontakt mit dem Kontrollvehikel erhalten wurden, mit den Ergebnissen der Messung, die nach dem Kontakt mit dem Arzneimittel erhalten wurden, und
Identifizieren des Ansprechens auf das Arzneimittel auf der Grundlage der Ergebnisse des Vergleichs, wobei eine Änderung der mRNA-Menge das Ansprechen des Patienten auf das Arzneimittel anzeigt.

## Revendications

1. °) Procédé de mesure de la sensibilité d'un patient atteint d'un cancer du sang à un médicament anti-cancer comprenant les étapes consistant à :
exposer le sang entier héparinisé du patient au médicament ex vivo pendant 7 heures ou moins,
exposer le sang entier héparinisé du patient à un véhicule de contrôle ex vivo pendant 7 heures ou moins,
après cette exposition, mesurer la quantité d'au moins un ARNm choisi dans le groupe formé par les ARNm codant pour un produit du gène PUMA et un produit de gène p21 à la fois dans les cellules sanguines exposées au médicament et dans les cellules sanguines exposées au véhicule de contrôle, et
identifier la sensibilité au médicament en comparant les résultats de mesure obtenus après exposition au véhicule de contrôle avec les résultats de mesure obtenus après l'exposition au médicament, une modification de quantité de ARNm indiquant la sensibilité du patient au médicament.

2. °) Procédé conforme à la revendication 1, selon lequel le véhicule de contrôle est choisi dans le groupe formé par les sels tamponnés au phosphate et le diméthyl sulfoxide.

3. °) Procédé conforme à la revendication 1, selon lequel le sang entier est stimulé pendant 5 heures ou moins.

4. °) Procédé conforme à la revendication 1, selon lequel le sang entier est stimulé pendant 2 à 4 heures.

5. °) Procédé conforme à la revendication 1, selon lequel l'effet du médicament est l'apoptose de cellules sanguines.

6. °) Procédé conforme à la revendication 1, selon lequel l'effet du médicament est un arrêt du cycle cellulaire dans des cellules sanguines.

7. °) Procédé conforme à la revendication 1, comprenant en outre une étape consistant à mesurer la quantité d'un second ARNm associé à un second effet du médicament dans des cellules sanguines, et selon lequel le premier effet du médicament est l'apoptose de cellules cellulaires et le premier ARNm code pour le produit du gène PUMA, et
le second effet du médicament est un arrêt du cycle cellulaire dans des cellules sanguines et le second ARNm code pour le produit du gène p21.

8. °) Procédé conforme à la revendication 5 ou 6, selon lequel le médicament est choisi dans le groupe formé par les médicaments suivants : étoposide, doxorubicin, fludarabine, mitoxantrone, rituximab, vindésine, pirarubicine, carboplatin, cyclophosphamide, bléomycin, vinblastine, vincristine, péplomycin, aclarubicin, daunorubicin, cisplatin, méthotrexate, 5-fluorouracil, cytarabine, dacarbazine, cyclophosphamide, et paclitaxel.

9. °) Procédé conforme aux revendications 5, 6, 7 ou 8, selon lequel le patient souffre de leucémie ou de lymphome leucémique.

10. °) Procédé conforme à la revendication 9, selon lequel la lectine est choisie dans le groupe formé par la phytohémagglutinine-P et le mitogène de la phytolague.

11. °) Procédé de mesure de la sensibilité de patients atteints de cancers du sang à un médicament choisi dans le groupe formé par les médicaments suivants : étoposide, doxorubicin, fludarabine, mitoxantrone, rituximab, vindésine, pirarubicin, carboplatin, cyclophosphamide, bléomycin, vinblastine, vincristine, péplomycin, aclarubicin, daunorubicin, cisplatin, méthotrexate, 5-fluorouracil, cytarabine, dacarbazine, cyclophosphamide, et paclitaxel comprenant les étapes consistant à :
exposer le sang entier héparinisé du patient au médicament ex vivo pendant 4 heures ou moins,
exposer le sang entier héparinisé du patient à un véhicule de contrôle ex vivo pendant 4 heures ou moins,
après cette exposition, mesurer la quantité d'au moins un ARNm choisi dans le groupe formé par les ARNm codant pour les produits du gène p21 et du gène PUMA dans des cellules sanguines,
comparer les résultats de mesure obtenus après l'exposition au véhicule de contrôle avec les résultats de mesure obtenus après exposition au médicament, et
identifier la sensibilité au médicament à partir des résultats de cette comparaison, une modification de la quantité d'ARNm indiquant une sensibilité du patient au médicament.


### SEQUENCE LISTING

<110> Hitachi Chemical Co., Ltd.
   Hitachi Chemical Research Center, Inc
   MITSUHASHI, Masato
<120> METHOD FOR TAILORING ADMINISTRATION OF
   DRUGS BY QUANTITATION OF mRNA
<130> HITACHI.066VPC
<150> 60/620,603 <151> 2004-10-20
<150> 60/653,557 <151> 2005-02-16
<150> 60/688,741 <151> 2005-06-08
<160> 52
<170> FastSEQ for Windows Version 4.0
<210> 1
   <211> 25
   <212> DNA
   <213> Artificial Sequence
<220>
   <223> Synthetic oligonucleotide primer sequence
<400> 1
   ttctgctgtc tctcctcaga tttct 25
<210> 2
   <211> 21
   <212> DNA
   <213> Artificial Sequence
<220>
   <223> Synthetic oligonucleotide primer sequence
<400> 2
   ggattagggc ttcctcttgg a 21
<210> 3
   <211> 22
   <212> DNA
   <213> Artificial Sequence
<220>
   <223> Synthetic oligonucleotide probe sequence
<400> 3
   ccactccaaa cgccggctga tc 22
<210> 4
   <211> 22
   <212> DNA
   <213> Artificial Sequence
<220>
   <223> Synthetic oligonucleotide primer sequence
<400> 4
   agaaccagga aacggaaaca ga 22
<210> 5
   <211> 20
   <212> DNA
   <213> Artificial Sequence
<220>
   <223> Synthetic oligonucleotide primer sequence
<400> 5
   tctccttcat gcgctgcttt 20
<210> 6
   <211> 25
   <212> DNA
   <213> Artificial Sequence
<220>
   <223> Synthetic oligonucleotide primer sequence
<400> 6
   gggtcagaga attgctgata atcat 25
<210> 7
   <211> 22
   <212> DNA
   <213> Artificial Sequence
<220>
   <223> Synthetic oligonucleotide primer sequence
<400> 7
   ccccgtttgt tcctgataaa ct 22
<210> 8
   <211> 18
   <212> DNA
   <213> Artificial Sequence
<220>
   <223> Synthetic oligonucleotide primer sequence
<400> 8
   tgcgctgctc tgccttct 18
<210> 9
   <211> 21
   <212> DNA
   <213> Artificial Sequence
<220>
   <223> Synthetic oligonucleotide primer sequence
<400> 9
   ccatgggtag cagctccttc t 21
<210> 10
   <211> 23
   <212> DNA
   <213> Artificial Sequence
<220>
   <223> Synthetic oligonucleotide primer sequence
<400> 10
   cacaggagaa tggataaggc aaa 23
<210> 11
   <211> 23
   <212> DNA
   <213> Artificial Sequence
<220>
   <223> Synthetic oligonucleotide primer sequence
<400> 11
   catccagcca gatttaggtt caa 23
<210> 12
   <211> 17
   <212> DNA
   <213> Artificial Sequence
<220>
   <223> Synthetic oligonucleotide primer sequence
<400> 12
   tccggcgcac cttctct 17
<210> 13
   <211> 23
   <212> DNA
   <213> Artificial Sequence
<220>
   <223> Synthetic oligonucleotide primer sequence
<400> 13
   cccaaagaca aagaaggcta caa 23
<210> 14
   <211> 21
   <212> DNA
   <213> Artificial Sequence
<220>
   <223> Synthetic oligonucleotide primer sequence
<400> 14
   catgtgtgtg gagagcgtca a 21
<210> 15
   <211> 21
   <212> DNA
   <213> Artificial Sequence
<220>
   <223> Synthetic oligonucleotide primer sequence
<400> 15
   gccggttcag gtactcagtc a 21
<210> 16
   <211> 20
   <212> DNA
   <213> Artificial Sequence
<220>
   <223> Synthetic oligonucleotide primer sequence
<400> 16
   gggcccagac tgtgaatcct 20
<210> 17
   <211> 24
   <212> DNA
   <213> Artificial Sequence
<220>
   <223> Synthetic oligonucleotide primer sequence
<400> 17
   acgtgctctc tctaaaccta tgca 24
<210> 18
   <211> 19
   <212> DNA
   <213> Artificial Sequence
<220>
   <223> Synthetic oligonucleotide probe sequence
<400> 18
   ccccgcccca tcaatccca 19
<210> 19
   <211> 20
   <212> DNA
   <213> Artificial Sequence
<220>
   <223> Synthetic oligonucleotide primer sequence
<400> 19
   ctcaggaggt gcacgtttca 20
<210> 20
   <211> 18
   <212> DNA
   <213> Artificial Sequence
<220>
   <223> Synthetic oligonucleotide primer sequence
<400> 20
   ttccaagggc acccatga 18
<210> 21
   <211> 19
   <212> DNA
   <213> Artificial Sequence
<220>
   <223> Synthetic oligonucleotide primer sequence
<400> 21
   gggagcccag agcttgaaa 19
<210> 22
   <211> 23
   <212> DNA
   <213> Artificial Sequence
<220>
   <223> Synthetic oligonucleotide primer sequence
<400> 22
   gcgctgtctt tactctccac ttc 23
<210> 23
   <211> 23
   <212> DNA
   <213> Artificial Sequence
<220>
   <223> Synthetic oligonucleotide primer sequence
<400> 23
   tccaggacag ttggatattg tca 23
<210> 24
   <211> 23
   <212> DNA
   <213> Artificial Sequence
<220>
   <223> Synthetic oligonucleotide primer sequence
<400> 24
   taaggagcag gcacagagaa aga 23
<210> 25
   <211> 22
   <212> DNA
   <213> Artificial Sequence
<220>
   <223> Synthetic oligonucleotide primer sequence
<400> 25
   aaatattccc cccaaggagt tc 22
<210> 26
   <211> 19
   <212> DNA
   <213> Artificial Sequence
<220>
   <223> Synthetic oligonucleotide primer sequence
<400> 26
   cgctcgtgtt cctcatgct 19
<210> 27
   <211> 22
   <212> DNA ,
   <213> Artificial Sequence
<220>
   <223> Synthetic oligonucleotide primer sequence
<400> 27
   tcctatggct ctgcaattgt ca 22
<210> 28
   <211> 21
   <212> DNA
   <213> Artificial Sequence
<220>
   <223> Synthetic oligonucleotide primer sequence
<400> 28
   ggcaggagtg aatggctctt c 21
<210> 29
   <211> 28
   <212> DNA
   <213> Artificial Sequence
<220>
   <223> Synthetic oligonucleotide primer sequence
<400> 29
   catacacttt ttctctttcc atgacatc 28
<210> 30
   <211> 20
   <212> DNA
   <213> Artificial Sequence
<220>
   <223> Synthetic oligonucleotide primer sequence
<400> 30
   gggcatcgca gtagcttctg 20
<210> 31
   <211> 22
   <212> DNA
   <213> Artificial Sequence
<220>
   <223> Synthetic oligonucleotide primer sequence
<400> 31
   caggcctatg caaaaagagg at 22
<210> 32
   <211> 18
   <212> DNA
   <213> Artificial Sequence
<220>
   <223> Synthetic oligonucleotide primer sequence
<400> 32
   cgcaccggaa gggaatct 18
<210> 33
   <211> 18
   <212> DNA
   <213> Artificial Sequence
<220>
   <223> Synthetic oligonucleotide primer sequence
<400> 33
   ggcaggcgac gagtttga 18
<210> 34
   <211> 27
   <212> DNA
   <213> Artificial Sequence
<220>
   <223> Synthetic oligonucleotide primer sequence
<400> 34
   gttcccatag agttccacaa aagtatc 27
<210> 35
   <211> 22
   <212> DNA
   <213> Artificial Sequence
<220>
   <223> Synthetic oligonucleotide primer sequence
<400> 35
   tcacatgctg gttgcttaat cc 22
<210> 36
   <211> 19
   <212> DNA
   <213> Artificial Sequence
<220>
   <223> Synthetic oligonucleotide primer sequence
<400> 36
   gcacaaggac cccatcaca 19
<210> 37
   <211> 21
   <212> DNA
   <213> Artificial Sequence
<220>
   <223> Synthetic oligonucleotide primer sequence
<400> 37
   cacggcagag aatgcctatg a 21
<210> 38
   <211> 20
   <212> DNA
   <213> Artificial Sequence
<220>
   <223> Synthetic oligonucleotide primer sequence
<400> 38
   cccaattgat gccactctca 20
<210> 39
   <211> 22
   <212> DNA
   <213> Artificial Sequence
<220>
   <223> Synthetic oligonucleotide primer sequence
<400> 39
   tttctgacgg caacttcaac tg 22
<210> 40
   <211> 18
   <212> DNA
   <213> Artificial Sequence
<220>
   <223> Synthetic oligonucleotide primer sequence
<400> 40
   ggtgcacagg gccttgag 18
<210> 41
   <211> 21
   <212> DNA
   <213> Artificial Sequence
<220>
   <223> Synthetic oligonucleotide probe sequence
<400> 41
   ccaaggccca gccctcacac a 21
<210> 42
   <211> 18
   <212> DNA
   <213> Artificial Sequence
<220>
   <223> Synthetic oligonucleotide primer sequence
<400> 42
   agccccctca ctcccaaa 18
<210> 43
   <211> 21
   <212> DNA
   <213> Artificial Sequence
<220>
   <223> Synthetic oligonucleotide primer sequence
<400> 43
   gggtgctgtg cttctgtgaa c 21
<210> 44
   <211> 22
   <212> DNA
   <213> Artificial Sequence
<220>
   <223> Synthetic oligonucleotide probe sequence
<400> 44
   ccactccaaa cgccggctga tc 22
<210> 45
   <211> 25
   <212> DNA
   <213> Artificial Sequence
<220>
   <223> Synthetic oligonucleotide primer sequence
<400> 45
   ttctgctgtc tctcctcaga tttct 25
<210> 46
   <211> 21
   <212> DNA
   <213> Artificial Sequence
<220>
   <223> Synthetic oligonucleotide primer sequence
<400> 46
   ggattagggc ttcctcttgg a 21
<210> 47
   <211> 19
   <212> DNA
   <213> Artificial Sequence
<220>
   <223> Synthetic oligonucleotide probe sequence
<400> 47
   ccccgcccca tcaatccca 19
<210> 48
   <211> 20
   <212> DNA
   <213> Artificial Sequence
<220>
   <223> Synthetic oligonucleotide primer sequence
<400> 48
   gggcccagac tgtgaatcct 20
<210> 49
   <211> 24
   <212> DNA
   <213> Artificial Sequence
<220>
   <223> Synthetic oligonucleotide primer sequence
<400> 49
   acgtgctctc tctaaaccta tgca 24
<210> 50
   <211> 27
   <212> DNA
   <213> Artificial Sequence
<220>
   <223> Synthetic oligonucleotide probe sequence
<400> 50
   ttgtcgccct tttctacttt gccagca 27
<210> 51
   <211> 22
   <212> DNA
   <213> Artificial Sequence
<220>
   <223> Synthetic oligonucleotide primer sequence
<400> 51
   tttctgacgg caacttcaac tg 22
<210> 52
   <211> 18
   <212> DNA
   <213> Artificial Sequence
<220>
   <223> Synthetic oligonucleotide primer sequence
<400> 52
   ggtgcacagg gccttgag 18